# EUROPEAN PATENT APPLICATION

(11) **EP 4 265 724 A1**
(43) Date of publication of application: **25.10.2023**
(21) Application number: 21907187.5
(22) Date of filing: 20.12.2021
(51) Int. Cl.: C12N 15/113, A61K 31/7088, A61K 48/00, A61P 31/20

(54) **RNAI AGENT FOR INHIBITING HBV EXPRESSION AND USE THEREOF**

(30) Priority: 18.12.2020 KR 20200178838
(71) Applicant: OliX Pharmaceuticals, Inc., Gyeonggi-do 16226 (KR)
(72) Inventor: HONG, Sun Woo, Yongin-si, Gyeonggi-do 16822 (KR); DUA, Pooja, Yongin-si, Gyeonggi-do 16928 (KR)
(74) Representative: Weickmann & Weickmann PartmbB
(86) International application number: PCT/KR2021/019408
(87) International publication number: WO 2022/131883

(57) **Abstract**

The present application relates to an RNA interference (RNAi) agent for inhibiting the expression of hepatitis B virus and uses thereof, and to an RNAi agent that inhibits the expression of hepatitis B virus and a pharmaceutical composition including the RNAi agent for amelioration or treatment of diseases caused by hepatitis B virus infection.

## Description

### Technical Field

The present application relates to an RNA interference (RNAi) agent for inhibiting the expression of hepatitis B virus (HBV) and uses thereof.

### Background Art

Hepatitis B is an infectious disease caused by infection with a hepatitis B virus (HBV), which causes malaise, loss of appetite, fatigue, nausea, vomiting, right upper abdominal discomfort during the cultivation period, and in severe cases, jaundice and pruritus. Globally, more than 2,000,000 people are infected with HBV annually, and while the development of HBV vaccines has reduced the prevalence of HBV to about 3 %, there are still about 1,250,000 carriers in Korea. The main obstacle to treating hepatitis B patients is the emergence of frequent medication resistance to HBV therapeutic agents, which requires the development of new therapeutic agents.

HBV is a 3,200 bp sized, incomplete, double-stranded DNA virus with an incomplete complementary structure of positive-stranded DNA inside a complete circular negative-stranded DNA. The HBV genome is surrounded by 4 open reading frames (ORFs), the longest of which is a polymerase ORF, which encodes a polymerase, followed by a surface ORF, which encodes a viral surface protein and is superimposed within the polymerase ORF. Meanwhile, a precore/core ORF and a core promoter/X ORF are partially overlapped with the polymerase ORF.

Proliferation of HBV with such a structure begins with the virus attaching to the surface of the hepatocyte and penetrating into the cytoplasm. Once inside the cytoplasm, the HBV nucleocapsid removes the envelope from the nuclear membrane and moves into the hepatocyte nucleus. Inside the nucleus, the HBV genome is converted by enzymes in the human body from an incomplete double-stranded relaxed circular (RC) DNA form to a covalently closed circular DNA (cccDNA) form with a complete double-stranded structure. This form serves as the most important template for transcribing all HBV messenger RNAs (mRNAs). Thereafter, 5 RNAs are transcribed from the cccDNA form of the viral chromosome. The transcription process is regulated by two enhancers, enhancer I located between S and X proteins, and enhancer II located just above a core promoter, and the enhancers I and II promote viral proliferation by upregulating the promoter. The transcripts produced by the 4 promoters are a pregenomic RNA of 3.5 kb, a precore RNA slightly larger than 3.5 kb, and subgenomic RNAs of 2.4 kb, 2.1 kb, and 0.7 kb. A set of promoters regulate the activity of transcripts, and RNA transcripts are translated to produce proteins. The 2.4 kb RNA transcript produces a preS1 protein, the 2.1 kb RNA transcript produces preS2 and S proteins, and the 0.7 kb transcript produces an X protein. Thereafter, the precore RNA produces HBeAg, and the pregenomic RNA produces the nucleocapsid of virus (core protein, HBcAg) and polymerase and also serves as the template for reverse transcription. Thereafter, in the cytoplasm, the encapsidation signal (epsilon, ε) of the pregenomic RNA and the polymerase react together to encapsidate the nucleocapsid (encapsidation), and negative strand DNA is generated by reverse transcription therein. At this time, direct repeat 1 (DR1), consisting of 11 nucleotides serves as the initiator of negative-stranded DNA. Once the negative-stranded DNA is produced as described above, DR2, which has an almost identical sequence to DR1, is initiated again at the 5' end of an opposite positive strand, and positive-stranded DNA begins to be produced, and RC DNA is formed as an incomplete but double-stranded DNA, and the nucleocapsid gradually matures. Thereafter, the nucleocapsid then re-enters the hepatocyte nucleus and gradually increases the amount of cccDNA in the liver, or forms an envelope membrane in a cytoplasmic trabeculae and is released from the hepatocyte in the form of a new virion through a Golgi apparatus.

On the other hand, treatment of diseases using RNA interference can treat diseases more safely since it targets mRNA and uses a small interfering RNA (siRNA) that regulates gene expression at a translational level. A siRNA consists of a sense strand with the same sequence as a target mRNA and an antisense strand with a complementary sequence to the sense strand. Conventional siRNA has a short duplex of 19 bp to 21 bp and 2 nucleotides protrude on 3' of both strands. Once inside a cell, siRNA attaches to a target mRNA, degrades the target mRNA, and inhibits the expression of a target gene. Since all mRNAs can be targeted by changing the sequence of the oligonucleotide, the expression of proteins with complex structures can be also inhibited, therefore enabling the treatment of diseases such as cancer, viral infections, and genetic diseases that are currently difficult to treat. However, siRNA introduced into a cell can cause side effects such as triggering immune responses and inhibiting non-target genes, and the delivery system for introducing siRNA into a cell is one of the biggest issues in developing a therapeutic agent using siRNA.

siRNA is negatively charged due to the phosphate backbone thereof, which repels the negatively charged cell membrane, and thus a delivery system is required to introduce the siRNA into the cell. One example of a delivery system is to wrap the siRNA with a positively charged liposome or polymer to offset the negative charge and introduce it into the cell. However, positively charged carriers can cause various side effects, such as unwanted toxicity due to adhesion to negatively charged cell membranes or formation of unwanted complexes through interaction with different types of protein in the cell. In addition, since siRNA is rapidly degraded by nucleases in the blood, the amount of siRNA that reaches a target cell may not be sufficient to significantly reduce the expression of a target gene. Therefore, a method to safely and effectively deliver siRNA into a target cell is needed.

Accordingly, the inventors of the present application have made intensive research efforts to develop siRNAs that target HBV, are delivered intracellularly without the aid of a delivery vehicle, and are highly resistant to nucleases, and as a result, by inhibiting transcripts for ORFs in the polymerase, S protein (surface antigen S), or HBx (X protein) regions of the HBV genome, siRNAs that can inhibit the proliferation of HBV is designed. Furthermore, the most efficient siRNAs are selected through efficacy screening, and it is confirmed that intracellular delivery issues can be overcome through modification, thereby completing the present disclosure.

The information in this Background Art section is intended solely to enhance the understanding of the background of the present disclosure and may not contain information that constitutes related art known to one of ordinary skill in the art to which the present disclosure belongs.

### Disclosure

### Technical Problem

An object of the present disclosure is to provide an RNA interference (RNAi) agent that inhibits hepatitis B virus (HBV) expression.

Another object of the present disclosure is to provide a pharmaceutical composition including the RNAi agent for ameliorating or treating a disease caused by HBV infection or a method of ameliorating or treating a disease caused by HBV infection.

### Technical Solution

In order to achieve the objectives, the present disclosure provides an RNA interference (RNAi) agent including: an antisense strand including a sequence complementary to a messenger RNA (mRNA) of a HBV S or HBV X gene of a HBV genome; and a sense strand forming a complementary binding with the antisense strand, wherein a 5' end of the antisense strand and a 3' end of the sense strand form a blunt end.

In addition, the present disclosure provides a pharmaceutical composition including the RNAi agent for ameliorating or treating a disease caused by HBV infection.

In addition, the present disclosure provides a method of ameliorating or treating a disease caused by HBV infection, the method including administering the RNAi agent to a subject.

### Advantageous Effects

According to the present disclosure, an asymmetric small interfering RNA (siRNA) that can efficiently inhibit the proliferation of hepatitis B virus (HBV) is provided. Furthermore, by the siRNA introduced into a cell without the aid of a delivery vehicle and chemically modified to be resistant to nucleases, HBV can be inhibited more efficiently in vivo, making it useful as a therapeutic agent for diseases caused by HBV infection.

### Description of Drawings

FIG. 1 schematically shows the structure of an asymmetric HBV asiRNA.
FIG. 2 shows the result of confirming the HBV proliferation inhibitory ability after treating HBV asiRNA to a Hela cell.
FIG. 3 shows the result of confirming the HBV proliferation inhibitory ability after treating a mouse hepatocyte with 4 types of HBV asiRNA.
FIG. 4 shows the result of confirming the HBV proliferation inhibitory ability after treating a mouse hepatocyte with 8 types of HBV cp-asiRNA.
FIG. 5 schematically shows a mouse model experimental procedure for evaluating the efficacy of HBV cp-asiRNA according to an embodiment.
FIG. 6 shows the result of confirming the HBV proliferation inhibitory ability after administering 2 types of HBV cp-asiRNA in mice.
FIG. 7 schematically shows an efficacy assay and duration assay experimental procedure in a mouse model for evaluating the efficacy of HBV cp-asiRNA according to an embodiment.
FIG. 8 shows the results of the efficacy assay of 21 types of HBV cp-asiRNA.
FIG. 9 shows the results of the duration assay of 21 types of HBV cp-asiRNA.
FIG. 10 schematically shows an experimental procedure in a cellular model for evaluating the efficacy of HBV cp-asiRNA according to an embodiment.
FIG. 11 shows the results of confirming the HBV proliferation inhibitory ability of HBV asiRNA and its cp-asiRNA
FIG. 12 schematically shows an experimental procedure in an adeno-associated virus (AAV)- hepatitis B virus (HBV) mouse model for evaluating the efficacy of HBV cp-asiRNA according to an embodiment.
FIG. 13 shows the result of confirming the HBV proliferation inhibitory ability of HBV cp-asiRNA in an AAV-HBV mouse model.
FIG. 14 shows the result of confirming the HBV proliferation inhibitory ability of HBV cp-asiRNA in an AAV-HBV mouse model by immunohistochemistry.
FIG. 15 shows the result of confirming the HBV proliferation inhibitory ability after treating a mouse hepatocyte with 20 types of HBV cp-asiRNA.
FIG. 16 schematically shows an efficacy assay and duration assay experimental procedure in a mouse model for evaluating the efficacy of HBV cp-asiRNA according to an embodiment.
FIG. 17 shows the results of the efficacy assay of 7 types of HBV cp-asiRNA.
FIG. 18 shows the results of the duration assay of 4 types of HBV cp-asiRNA.
FIG. 19 shows the IC50 test results of 3 types of HBV cp-asiRNA.
FIG. 20 shows the results of confirming the HBV proliferation inhibitory ability of 3 types of HBV cp-asiRNA with E-vinylphosphonate (E-VP) modifications.

### Mode for Invention

Unless otherwise defined, all technical and scientific terms used herein have the same meaning as would be commonly understood by a person skilled in the art to which the present disclosure belongs. In general, the nomenclature used herein is well known and in common use in the art.

The following are definitions of key terms used in the detailed description of the present disclosure and elsewhere.

The term "RNA interference (RNAi)" refers to a mechanism for inhibiting the expression of a target gene by introducing double-stranded RNA (dsRNA), which consists of a strand with a sequence homologous to the messenger RNA (mRNA) of the target gene and a strand with a sequence complementary to it, into a cell to induce degradation of the target gene mRNA.

The term "nucleic acid molecules inducing RNAi", "RNAi agent", or "agent for inducing RNAi" refers to any nucleic acid molecule capable of inhibiting or downregulating gene expression or viral replication by mediating the RNA interference in a sequence-specific manner. This term may refer to an individual nucleic acid molecule, a plurality of the nucleic acid molecules, or a pool of the nucleic acid molecules. In an embodiment, the nucleic acid molecule for inducing RNAi may be a small interfering RNA (siRNA).

The term "small interfering RNA (siRNA)" refers to a short dsRNA that mediates efficient gene silencing in a sequence-specifically manner.

The term "antisense strand" refers to a polynucleotide that is substantially or 100 % complementary to a target nucleic acid of interest, for example, the antisense strand may be complementary in whole or in part to a mRNA, a non-mRNA RNA sequence (for example, microRNA, piwiRNA, tRNA, rRNA, and hnRNA), or a coding or non-coding DNA sequence.

The term "sense strand" refers to a polynucleotide having the same nucleic acid sequence as a target nucleic acid, for example, the sense strand is identical in whole or in part to a mRNA, a non-mRNA RNA sequence (for example, microRNA, piwiRNA, tRNA, rRNA, and hnRNA), or a coding or non-coding DNA sequence.

The term "gene" is considered in a broadest sense, and may encode a structural protein or a regulatory protein. In this context, regulatory proteins include transcription factors, heat shock proteins, or proteins involved in DNA/RNA replication, transcription, and/or translation. In the present disclosure, a target gene to be inhibited is inherent in a viral genome, and may be incorporated into an animal gene or present as an extrachromosomal component. For example, a target gene may be a gene in a HBV genome. In this case, a siRNA molecule is useful for inactivating translation of the HBV gene in a mammalian cell.

The term "hepatitis B virus (HBV)" is a virus belonging to the Hepadnaviridae family and infiltrates hepatocytes, causing acute and chronic hepatitis, etc. The HBV undergoes processes of: attaching to the surface of a hepatocyte to penetrate into the cell; shedding off an outer shell thereof in the cytoplasm, entering the nucleus, and proliferating DNA replication; assembling a complete virus form thereof by accumulating a shell in the cytoplasm; being released outside the hepatocyte; and attaching again to another hepatocyte for further proliferation repeatedly. Specifically, covalently closed circular DNA (cccDNA) is present in the nucleus of HBV-infected hepatocytes and serves as a template for HBV transcription, and inhibiting the transcription and translation process of genes mediating this process may be effective in treating diseases caused by HBV infection.

In particular, an RNAi agent according to an aspect targeting a single HBV gene can lead to significant inhibition of most or all HBV transcript expression, as the transcription of the HBV genome leads to polycistronic overlapping RNA. For example, an RNAi agent targeting an S gene of a HBV genome may lead to the inhibition of not only S gene expression but also expression of "downstream" genes, and an RNAi agent targeting an X gene of the HBV genome may lead to the inhibition of not only X gene expression but also expression of "downstream" genes. Therefore, by inhibiting the expression of either the HBV S or HBV X genes of the HBV genome, replication or proliferation of the HBV can be inhibited. In an embodiment, inhibition of transcripts for ORFs of the S protein (surface antigen S) or HBx (X protein) region among the HBV genome may be achieved in a region overlapping with the ORFs of the polymerase region of the HBV genome.

The objectives of the present disclosure are twofold: first, to design and screen siRNAs targeting the HBV gene and to identify those that most efficiently inhibit the replication or proliferation of HBV, and second, to introduce chemical modifications to deliver the siRNA into the cell without a specific carrier and to increase the resistance to a nuclease. In order to pass through cell membranes composed of phospholipids, siRNA needs to be small or hydrophobic. However, siRNA is negatively charged by the phosphate backbone, making it difficult to penetrate cell membranes. In addition, resistance to nucleases should be increased to have a long lifetime in serum so that the amount reached to the target is sufficient to induce effective RNAi. Therefore, modifications have been introduced to overcome the delivery issues of siRNA.

According to an embodiment of the present disclosure, an asiRNA targeting a S or X gene of a HBV genome was first designed, and an asiRNA with excellent HBV proliferation inhibitory ability was screened in a cell transfected with HBV plasmid. Thereafter, the following four kinds of modifications were introduced into the selected siRNA to modify the asiRNA to have cell-penetrating ability and resistance to nucleases. First, N-acetyl-galactosamine (GaINAc) derivatives are bound to the 3' end of the sense strand to enable delivery to the liver tissue. Second, the phosphate backbone near the 5' end or 3' end of the sense strand or antisense strand is substituted with phosphorothioate to have resistance to exogenous nuclease, enabling uptake into cells and bioavailability of siRNA in vivo. Third, 2' of the sugar is modified with OMethyl to give resistance to nuclease, lowers siRNA immunogenicity, and reduces off-target effects. Fourth, 2' of the sugar is modified with fluoro to give stability to the double strand duplex, increasing stability in serum and enabling efficient silencing in vitro and in vivo. By applying these modifications to the siRNA, the siRNA gains cell-penetrating ability and stays in the serum longer, enabling more efficient gene silencing as a sufficient amount of siRNA is delivered to the target cell.

Therefore, in an aspect, the present disclosure relates to an RNAi agent including a sense strand and an antisense strand, which inhibits the expression of HBV, wherein the antisense strand has a length of 19 nt to 23 nt; wherein the sense strand has a length of 15 nt to 17 nt, forms a complementary bond with the antisense strand, and including a sequence selected from a group consisting of SEQ ID NOs: 1, 3, 5, 7, 9, 11, 13, 15, 17, 19, 37, 39, 41, 43, 45, 47, 49, 51, and 53, and includes a polyvalent galactose or N-acetyl-galactosamine derivative; and an RNAi agent wherein the 5' end of the antisense strand and the 3' end of the sense strand form a blunt end.

In addition, in an aspect, the present disclosure relates to an RNAi agent including a sense strand and an antisense strand, which inhibits the expression of HBV, wherein the antisense strand has a length of 19 nt to 23 nt; wherein the sense strand has a length of 15 nt to 17 nt, forms a complementary bond with the antisense strand, and includes a sequence selected from the group consisting of SEQ ID NOs: 21, 23, 25, 27, 29, 31, 33, 35, 55, 57, 59, 61, 63, 65, 67, 69, 71, 73, and 75, and includes a polyvalent galactose or N-acetyl-galactosamine derivative; and an RNAi agent wherein the 5' end of the antisense strand and the 3' end of the sense strand form a blunt end.

In addition, in an aspect, the present disclosure relates to a pharmaceutical composition including a RNAi agent for ameliorating or treating a disease caused by HBV infection; wherein the RNAi agent including a sense strand forming a complementary bond with a antisense strand, wherein the antisense strand including a sequence complementary to an overlapping open reading frame (ORF) encoding HBV polymerase and surface antigen S or an overlapping ORF encoding HBV polymerase and X protein (HBx).

In the present disclosure, siRNA is a concept that includes any substance that has a general RNAi action. RNAi is an intracellular gene regulation mechanism first discovered in Caenorthabditis elegans in 1998. The mechanism of action is known that an antisense strand among a RNA duplexes introduced into a cell complementarily binds to an mRNA of a target gene to induce degradation of a target gene. Among them, siRNA is one of the methods of inhibiting gene expression "in vitro". Theoretically, siRNA of 19 bp to 21 bp can selectively inhibit almost any gene, and thus can be developed as a therapeutic agent for various gene-related diseases such as cancer and viral infections, and is currently the most promising candidate art for medication development. The first attempt of in vivo treatment using siRNA in a mammal was in mid-2003, and since then, numerous reports on in vivo treatment have been made with many attempts for applied research.

However, despite the promise, side effects and disadvantages of siRNA continue to be reported. The development of RNAi-based therapeutic agents requires overcoming barriers such as 1) lack of effective delivery systems, 2) off-target effects, 3) induction of immune responses, and 4) saturation of intracellular RNAi machinery. Although siRNA is an effective way to directly regulate the expression of target genes, these issues have hindered the development of therapeutic agents. In this regard, asymmetric siRNA(asymmetric shorter duplex siRNA, asiRNA) is an asymmetric RNAi- derived structure with a shorter double helix length compared to the 19+2 structure of conventional siRNA. This technique overcomes issues such as off-target effects, saturation of the RNAi mechanism, and immune response by toll-like receptor 3 (TLR3) identified in the relating siRNA structure arts, and enables the development of RNAi medications with low side effects.

Based on this, an embodiment presents an asymmetric siRNA including a sense strand and an antisense strand complementary to the sense strand, wherein a siRNA according to an embodiment can effectively inhibit expression of the HBV target gene to the desired extent without causing off-target effects, saturation of the RNAi mechanism, etc. while maintaining a stable high delivery efficiency.

In the present disclosure, the RNAi agent may be identified in that the sense strand has a length of 15 nt to 17 nt and the antisense strand has a length of 18 nt or more. Although not limited thereto, the antisense strand may be identified as having a length of 18 nt to 31 nt, and preferably as having a length of 18 nt to 23 nt. More preferably, the sense strand may be identified as having a length of 16 nt or 17 nt and the complementary antisense strand may be identified as having a length of 19 nt, 20 nt, 21 nt or 22 nt, but is not limited thereto.

The 3' end of the sense strand and the 5' end of the antisense strand form a blunt end. The 3' end of the antisense strand may include an overhang of, for example, 1 nt to 16 nt.

According to an embodiment of the present disclosure, HBV asiRNA targeting S or X gene among the HBV genomes was designed, and the inhibitory efficacy of infected HBV was confirmed in HBV-transfected cells or transient cells expressing transiently HBV.

In an RNAi agent according to the present disclosure, a sense strand may be selected from a group consisting of SEQ ID NOs: 1, 3, 5, 7, 9, 11, 13, 15, 17, 19, 37, 39, 41, 43, 45, 47, 49, 51, and 53, and more preferably may be SEQ ID NOs: 7, 13, 29, 33, 65, or 67.

In an RNAi agent according to the present disclosure, an antisense strand may be selected from a group consisting of SEQ ID NOs: 2, 4, 6, 8, 10, 12, 14, 16, 18, 20, 38, 40, 42, 44, 46, 48, 50, 52, and 54, and may preferably be SEQ ID NOs: 8, 14, 30, 34, or 66.

In other RNAi agent according to the present disclosure, a sense strand may be selected from a group consisting of SEQ ID NOs: 21, 23, 25, 27, 29, 31, 33, 35, 55, 57, 59, 61, 63, 65, 67, 69, 71, 73, and 75, and may preferably be SEQ ID NOs: 29, 33, 65, 67, 73, or 75.

In other RNAi agent according to the present disclosure, an antisense strand may be selected from a group consisting of SEQ ID NOs: 22, 24, 26, 28, 30, 32, 34, 36, 56, 58, 60, 62, 64, 66, 68, 70, 72, 74, and 76, and may preferably be SEQ ID NOs: 30, 34, 66, 68, 74, or 76.

In the present disclosure, the sense strand or antisense strand of the RNAi agent may include one or more chemical modifications.

A typical siRNA is unable to pass through cell membranes due to high negative charge due to the phosphate backbone structure and high molecular weight, and are rapidly degraded and cleared from the blood, making it difficult to deliver sufficient amounts to induce RNAi at an actual target site. Currently, many high-efficiency delivery methods using cationic lipids and cationic polymers have been developed for in vitro delivery, but in the case of in vivo, it is difficult to deliver siRNA with the same high efficiency as in vitro, and the efficiency of siRNA delivery is reduced by interaction with various proteins present in vivo.

Therefore, by introducing chemical modifications to the asymmetric siRNA structure, the inventors of the present application developed a self-delivering asiRNA construct (cp-asiRNA) that can be effectively and efficiently delivered into cells without a separate delivery vehicle.

In the present disclosure, the sense strand may include a trivalent GaINAc derivatives.

In the present disclosure, the chemical modification in the sense strand or antisense strand may include one or more selected from a group consisting of the following: substitution of an -OH group at the 2' carbon position of a sugar structure in the nucleotide with -CH₃ (methyl), -OCH₃ (methoxy), -NH₂ , -F (fluoro), -O-2- methoxyethyl - O-propyl, -O-2-methylthioethyl, -O-3-aminopropyl, or -O-3- dimethylaminopropyl; substitution of an oxygen in the sugar structure in the nucleotide with sulfur; modification of the nucleotide bond to a phosphorothioate, boranophosphate, or methyl phosphonate; modification to peptide nucleic acid (PNA), locked nucleic acid (LNA), or unlocked nucleic acid (UNA) form; and linkage to a phosphate group, E-vinylphosphonate, or cell-penetrating peptide.

In an embodiment, the antisense strand may include one or more chemical modifications selected from the following: modification of 3 to 5 nucleotide bonds adjacent to the 3' end or 5' end to a phosphorothioate, boranophosphate, or methyl phosphonate; substitution of an -OH group at the 2' carbon position of a sugar structure in 2 or more nucleotides with -CH₃ (methyl), -OCH₃ (methoxy), -NH₂ , -F (fluoro), -O2-methoxyethyl - O-propyl, -O-2-methylthioethyl, -O-3-aminopropyl, or-O-3- dimethylaminopropyl; and the addition of a phosphate group, E-vinylphosphonate, or cell-penetrating peptide at the 5' end.

In an embodiment, the sense strand may include one or more chemical modifications selected from the following: modification of 2 to 4 nucleotide bonds adjacent to the 5' end to a phosphorothioate, boranophosphate, or methyl phosphonate; substitution of an -OH group at the 2' carbon position of a sugar structure in 2 or more nucleotides to -CH₃ (methyl), -OCH₃ (methoxy), -NH₂ , -F (fluoro), -O-2-methoxyethyl - O-propyl, -O-2-methylthioethyl, -O-3-aminopropyl, or -O-3-dimethylaminopropyl; and the addition of a cell-penetrating peptide at the 3' end.

In an embodiment, a modification in which an -OH group is substituted with -OCH₃ (methoxy) or -F (fluoro) at the 2' carbon position of a sugar structure in 2 or more nucleotides of the sense or antisense strand; a modification in which 10 % or more nucleotide bonds in the sense or antisense strand are phosphorothioate; and at least one modification selected from the group consisting of a phosphate group linkage or an E-vinylphosphonate linkage at the 5' end of the antisense strand.

In an RNAi agent according to the present disclosure, an antisense strand may be selected from a group consisting of (a) to (d) of Table 1, and the sense strand may be selected from the group consisting of (e) to (h) of Table 1. Preferably, the antisense strand may be any one of (a) or (c) of Table 1, and the sense strand may be any one of (e) or (g) of Table 1.

**Table 1**

| | Sequence (5' > 3') |
|---|---|
| (a) | P- mU*fA*mCfGmAfAmCfCmAfCmUmGmAfAmC*fA*mA*fA*m U |
| (b) | P- mU*fA*mCmGmAfAmCfCfAmCmUmGmAfAmC*fA*mA*mA* mU |
| (c) | P- mU*fA*mGfAmUfGmAfGmAfAmGmGmCfAmC*fA*mG*fA*m C |
| (d) | P- mU*fA*mGmAmUfGmAfGfAmAmGmGmCfAmC*fA*mG*mA* mC |
| (e) | mU*fG*mUfUfCfAmGfUmGfGmUfUmCfGmUfA-GalNAc |
| (f) | mU*fG*mUfUfCfAmGmUmGmGmUmUmCmGmUmA- GalNAc |
| (g) | mU*fG*mUfGfCfCmUfUmCfUmCfAmUfCmUfA-GalNAc |
| (h) | mU*fG*mUfGfCfCmUmUmCmUmCmAmUmCmUmA- GalNAc |

In the sequence above, * refers to a phosphorothioated bond, m refers to a 2'-O-methyl, 2'-F- refers to a 2'-fluoro, P refers to a 5'-phosphate group, and GalNAc refers to an N-acetyl-galactosamine derivative linked thereto..

In other RNAi agent according to the present disclosure, an antisense strand may be any one selected from a group consisting of (a1) to (u1) in Table 2, and a sense strand may be any one selected from a group consisting of (a2) to (u2) in Table 2. Preferably, the antisense strand may be any one of (a1), (b1), (j1), (q1), (s1), (t1) or (u1) of Table 2 below, and the sense strand may be any one of (a2), (b2), (j2), (q2), (s2), (t2) or (u2) of Table 2 below. More preferably, the antisense strand may be any one of (a1), (j1), (s1), (t1), or (u1) of Table 2, and the sense strand may be any one of (a2), (j2), (s2), (t2), or (u2) of Table 2.

**Table 2**

| No. | Sequence (5' > 3') | Sequence Name |
|---|---|---|
| (a1) | P- mU*fA*mGfAmGfGmAfCmAfAmAmCmGfGmG*fC*mA *fA*mC | OLX703A-020-1_antisense |
| (b1) | P- mU*fA*mGfAmGfGmAfCmAfAmAmCmGfGmG*fC*mA *fA*mC | OLX703A-020-3_antisense |
| (c1) | P- mU*fA*mGmAmGfGmAmCmAmAmAmCmGfGmG*fC *mA*mA*mC | OLX703A-020-5_antisense |
| (d1) | EVP- mU*fA*mGfAmGfGmAfCmAfAmAmCmGfGmG*fC*mA *fA*mC | OLX703A-020-17_antisense |
| (e1) | P- mU*fA*mCmGmAfAmCmCmAmCmUmGmAfAmC*fA* mA*mA*mU | OLX703A-032-5_antisense |
| (f1) | P- mU*fA*mCfGmAfAmCfCmAfCmUmGmAfAmC*fA*mA* fA*mU | OLX703A-032-9_antisense |
| (g1) | P- mU*fG*mAmGmAfAmGfGfCmAmCmAmGfAmC*fG*m G*mG*mG | OLX703A-044-2_antisense |
| (h1) | P- mU*fG*mAfGmAfAmGfGmCfAmCmAmGfAmC*fG*mG *fG*mG | OLX703A-044-3_antisense |
| (i1) | P- mU*fG*mAfGmAfAmGfGmCfAmCmAmGfAmC*fG*mG *fG*mG | OLX703A-044-13_antisense |
| (j1) | P- mU*fC*mAfGmAfUmGfAmGfAmAmGmGfCmA*fC*mA *fG*mA | OLX703A-046-3_antisense |
| (k1) | P- mU*fC*mAmGmAmUfGmAfGmAmAmGmGfCmA*fC* mA*mG*mA | OLX703A-046-8_antisense |
| (l1) | P- mU*fC*mAfGmAfUmGfAmGfAmAmGmGfCmA*fC*mA *fG*mA | OLX703A-046-13_antisense |
| (m1) | EVP- mU*fC*mAfGmAfUmGfAmGfAmAmGmGfCmA*fC*mA *fG*mA | OLX703A-046-17_antisense |
| (n1) | P- mU*fA*mUmGmAmGfAmAfGmGmCmAmCfAmG*fA* mC*mG*mG | OLX703A-080-6_antisense |
| (o1) | P- mU*fA*mUmGmAmGfAmAfGmGmCmAmCfAmG*fA* mC*mG*mG | OLX703A-080-16_anti sense |
| (p1) | P- mU*fA*mGfAmUfGmAfGmAfAmGmGmCfAmC*fA*mG *fA*mC | OLX703A-082-1_antisense |
| (q1) | P- mU*fA*mGmAmUfGmAfGfAmAmGmGmCfAmC*fA*m G*mA*mC | OLX703A-082-2_antisense |
| (r1) | P- mU*fA*mGmAmUfGmAmGmAmAmGmGmCfAmC*fA* mG*mA*mC | OLX703A-082-5_antisense |
| (s1) | P- mU*fA*mGmAmUmGfAmGfAmAmGmGmCfAmC*fA* mG*mA*mC | OLX703A-082-12_antisense |
| (t1) | P- mU*fA*mGmAmUmGfAmGfAmAmGmGmCfAmC*fA* mG*mA*mC | OLX703A-082-16_antisense |
| (u1) | EVP- mU*fA*mGmAmUmGfAmGfAmAmGmGmCfAmC*fA* mG*mA*mC | OLX703A-082-17_antisense |
| (a2) | mG*fC*mCfCfGfUmUfUmGfUmCfCmUfCmUfA- GalNAc | OLX703A-020-1_sense |
| (b2) | mG*fC*mCfCfGfUmUmUmGmUmCmCmUmCmUmA- GalNAc | OLX703A-020-3_sense |
| (c2) | mG*fC*mCfCfGfUmUfUmGfUmCfCmUfCmUfA- GalNAc | OLX703A-020-5_sense |
| (d2) | mG*fC*mCfCfGfUmUfUmGfUmCfCmUfCmUfA- GalNAc | OLX703A-020-17_sense |
| (e2) | mU*fG*mUfUfCfAmGfUmGfGmUfUmCfGmUfA- GalNAc | OLX703A-032-5_sense |
| (f2) | mU*fG*mUfUfCfAmGmUmGmGfUfUmCmGmUmA- GalNAc | OLX703A-032-9_sense |
| (g2) | mC*fG*mUfCfUfGmUfGmCfCmUfUmCfUmCfA- GalNAc | OLX703A-044-2_sense |
| (h2) | mC*fG*mUfCfUfGmUmGmCmCmUmUmCmUmCmA- GalNAc | OLX703A-044-3_sense |
| (i2) | mC*fG*mUfCfUfGmUmGmCmCmUmUmCfUmCfA- GalNAc | OLX703A-044-13_sense |
| (j2) | mG*fU*mGfCfCfUmUmCmUmCmAmUmCmUmGmA- GalNAc | OLX703A-046-3_sense |
| (k2) | mG*fU*mGfCfCfUmUmCmUmCmAmUmCmUmGmA- GalNAc | OLX703A-046-8_sense |
| (l2) | mG*fU*mGfCfCfUmUmCmUmCmAmUmCfUmGfA- GalNAc | OLX703A-046-13_sense |
| (m2) | mG*fU*mGfCfCfUmUmCmUmCmAmUmCmUmGmA- GalNAc | OLX703A-046-17_sense |
| (n2) | mU*fC*mUfGfUfGmCfCmUfUmCfUmCfAmUfA- GalNAc | OLX703A-080-6_sense |
| (o2) | mU*fC*mUfGfUfGmCmCmUmUmCmUmCfAmUfA- GalNAc | OLX703A-080-16_sense |
| (p2) | mU*fG*mUfGfCfCmUfUmCfUmCfAmUfCmUfA- GalNAc | OLX703A-082-1_sense |
| (q2) | mU*fG*mUfGfCfCmUfUmCfUmCfAmUfCmUfA- GalNAc | OLX703A-082-2_sense |
| (r2) | mU*fG*mUfGfCfCmUfUmCfUmCfAmUfCmUfA- GalNAc | OLX703A-082-5_sense |
| (s2) | mU*fG*mUfGfCfCmUmUmCmUfCfAmUmCmUmA- GalNAc | OLX703A-082-12_sense |
| (t2) | mU*fG*mUfGfCfCmUmUmCmUmCmAmUfCmUfA- GalNAc | OLX703A-082-16_sense |
| (u2) | mU*fG*mUfGfCfCmUmUmCmUfCfAmUmCmUmA- GalNAc | OLX703A-082-17_sense |

In the sequence above, * refers to a phosphorothioated bond, m refers to a 2'-O-methyl, 2'-F- refers to a 2'-fluoro, P refers to a a 5'-phosphate group, EVP refers to a 5' E-vinylphosphonate, and GalNAc referes to an N-acetyl-galactosamine derivative linked thereto..

In another RNAi agent according to the present disclosure, an antisense strand may be selected from a group consisting of (a1) to (u1) of Table 3, and a sense strand may be selected from a group consisting of (a2) to (u2) of Table 3. Preferably, the antisense strand is (e1), (k1), (11), (n1) or (vl) of Table 3, and the sense strand is (e2), (k2), (l2), (n2) or (v2) of Table 3. More preferably, the antisense strand is (k1), (11), (n1) or (v1) of Table 3, and the sense strand is (k2), (l2), (n2) or (v2) of Table 3.

**Table 3**

| No | Sequence (5' > 3') | Sequence Name |
|---|---|---|
| (a1) | P- mU*fA*mGmAmUmGfAmGfAmAmGmGmCfAmCfAmGmA* mC*fG*mG | OLX703A-103-16_antisense |
| (b1) | P- mU*fA*mGmAmUfGmAfGfAmAmGmGmCfAmC*fA*mG*m A*mC*mG*mG | OLX703A-103-24_antisense |
| (c1) | P- mU*fA*mGmAmUfGmAfGfAmAmGmGmCfAmCfAmG*mA* mC*fG*mG | OLX703A-103-30_antisense |
| (d1) | P- mU*fA*mGmAmUfGmAfGfAmAmGmGmCfAmCfAmG*mA* mC*mG*mG | OLX703A-103-47_antisense |
| (e1) | P- mU*fA*mGmAmUfGmAfGfAmAmGmGmCfAmC*fA*mG*m A*mC*fG*mG | OLX703A-103-49_antisense |
| (f1) | P- mU*fA*mGmAmUfGmAfGfAmAmGmGmCfAmC*fA*mG*m A*mC*mG*mG | OLX703A-103-67_antisense |
| (g1) | P- mU*fA*mGmAmUfGmAfGfAmAmGmGmCfAmC*fA*mG*m A*mC*fG*mG | OLX703A-103-71_antisense |
| (h1) | P- mU*fA*mGmAmUmGfAmGfAmAmGmGmCfAmC*fA*mG*f A*mC*mG*mG | OLX703A-103-82_antisense |
| (i1) | P- mU*fA*mGmAmUmGfAmGfAmAmGmGmCfAmCfA*mG*m A*mC*fG*mG | OLX703A-103-85_antisense |
| (j1) | P- mU*fA*mGmAmUmGfAmGfAmAmGmGmCfAmCfA*mG*fA *mC*fG*mG | OLX703A-103-89_antisense |
| (k1) | P- mU*fA*mGmAmUfGmAfGfAmAmGmGmCfAmC*fA*mG*m A*mC*mG*mG | OLX703A-103-91_antisense |
| (l1) | EVP- mU*fA*mGmAmUfGmAfGfAmAmGmGmCfAmC*fA*mG*m A*mC*mG*mG | OLX703A-103-101_antisense |
| (m1 ) | P- mU*fA*mGmAmUmGfAmGfAmAmGmGmCfAmCfA*mG*m A*mC*mG*Mg | OLX703A-107-2_antisense |
| (n1) | P- mU*fA*mGmAmUfGmAfGfAmAmGmGmCfAmCfAmG*mA* mC*fG*mG | OLX703A-107-19_antisense |
| (o1) | P- mU*fA*mGmAmUmGfAmGfAmAmGmGmCfAmCfA*mG*m A*mC*fG*mG | OLX703A-107-27_antisense |
| (p1) | P- mU*fA*mGmAmUmGfAmGfAmAmGmGmCfAmCfAmG*fA* mC*fG*mG | OLX703A-107-29_antisense |
| (q1) | P- mU*fA*mGmAmUfGmAfGfAmAmGmGmCfAmCfAmG*mA* mC*fG*mG | OLX703A-107-35_antisense |
| (r1) | P- mU*fA*mGmAmUmGfAmGfAmAmGmGmCfAmCfAmG*mA *mC*mG*mG | OLX703A-107-39_antisense |
| (s1) | P- mU*fA*mGmAmUmGfAmGfAmAmGmGmCfAmCfA*mG*m A*mC*fG*mG | OLX703A-107-45_antisense |
| (t1) | P- mU*fA*mGmAmUmGfAmGfAmAmGmGmCfAmCfA*mG*fA *mC*fG*mG | OLX703A-107-48_antisense |
| (u1) | P- mU*fA*mGmAmUfGmAfGfAmAmGmGmCfAmCfAmG*mA* mC*fG*mG | OLX703A-107-53_antisense |
| (v1) | EVP- mU*fA*mGmAmUfGmAfGfAmAmGmGmCfAmCfAmG*mA* mC*fG*mG | OLX703A-107-55_antisense |
| (a2) | mU*fG*mUfGfCfCmUfUmCfUmCfAmUfCmUfA-GalNAc | OLX703A-103-16_sense |
| (b2) | mU*fG*mUfGfCfCmUfUmCfUmCfAmUfCmUfA-GalNAc | OLX703A-103-24_sense |
| (c2) | mU*fG*mUfGfCfCmUfUmCfUmCfAmUfCmUfA-GalNAc | OLX703A-103-30_sense |
| (d2) | mU*fG*mUfGfCfCmUmUmCmUfCfAmUmCmUmA-GalNAc | OLX703A-103-47_sense |
| (e2) | mU*fG*mUfGfCfCmUmUmCmUfCfAmUmCmUmA-GalNAc | OLX703A-103-49_sense |
| (f2) | mU*fG*mUfGfCfCmUmUmCmUfCmAmUmCmUmA- GalNAc | OLX703A-103-67_sense |
| (g2) | mU*fG*mUfGfCfCmUmUmCmUfCmAmUmCmUmA- GalNAc | OLX703A-103-71_sense |
| (h2) | mU*fG*mUfGfCfCmUmUmCmUmCfAmUmCmUmA- GalNAc | OLX703A-103-82_sense |
| (i2) | mU*fG*mUfGfCfCmUmUmCmUmCfAmUmCmUmA- GalNAc | OLX703A-103-85_sense |
| (j2) | mU*fG*mUfGfCfCmUmUmCmUmCfAmUmCmUmA- GalNAc | OLX703A-103-89_sense |
| (k2) | mU*fG*mUfGfCfCmUmUmCmUmCfAmUmCmUmA- GalNAc | OLX703A-103-91_sense |
| (l2) | mU*fG*mUfGfCfCmUmUmCmUmCfAmUmCmUmA- GalNAc | OLX703A-103-101_sense |
| (m2 ) | mC*mU*fGmUfGfCfCmUmUmCmUfCfAmUmCmUmA- GalNAc | OLX703A-107-2_sense |
| (n2) | mC*mU*fGmUfGfCfCmUmUmCmUfCfAmUmCmUmA- GalNAc | OLX703A-107-19_sense |
| (o2) | mC*mU*fGmUfGfCfCmUmUmCmUfCmAmUmCmUmA- GalNAc | OLX703A-107-27_sense |
| (p2) | mC*mU*fGmUfGfCfCmUmUmCmUfCmAmUmCmUmA- GalNAc | OLX703A-107-29_sense |
| (q2) | mC*mU*fGmUfGfCfCmUmUmCmUfCmAmUmCmUmA- GalNAc | OLX703A-107-35_sense |
| (r2) | mC*mU*fGmUfGfCfCmUmUmCmUmCfAmUmCmUmA- GalNAc | OLX703A-107-39_sense |
| (s2) | mC*mU*fGmUfGfCfCmUmUmCmUmCfAmUmCmUmA- GalNAc | OLX703A-107-45_sense |
| (t2) | mC*mU*fGmUfGfCfCmUmUmCmUmCfAmUmCmUmA- GalNAc | OLX703A-107-48_sense |
| (u2) | mC*mU*fGmUfGfCfCmUmUmCmUmCfAmUmCmUmA- GalNAc | OLX703A-107-53_sense |
| (v2) | mC*mU*fGmUfGfCfCmUmUmCmUfCfAmUmCmUmA- GalNAc | OLX703A-107-55_sense |

In another aspect, the present disclosure relates to a pharmaceutical composition including the RNAi agent for ameliorating or treating a disease caused by HBV infection.

In the present disclosure, a disease caused by HBV infection refers to a disease or disorder caused by, or associated with, HBV infection and/or replication, and may include any disease, disorder, or pathology that would benefit from a reduction in HBV gene expression and/or replication. The disease may be, for example, but are not limited to, hepatitis D virus infection, delta hepatitis, acute hepatitis B, acute fulminant hepatitis B, chronic hepatitis B, liver fibrosis, liver cirrhosis, liver failure, or liver cancer.

In addition to the RNAi agent which is the active ingredient, the pharmaceutical composition may be prepared to include one or more pharmaceutically acceptable carriers. The pharmaceutically acceptable carrier should be compatible with the active ingredient of the present disclosure and may be saline, sterile water, ringer's solution, buffered saline, dextrose solution, maltodextrin solution, glycerol, ethanol, and mixtures of one or more of these components, and other common additives such as antioxidants, buffers, bacteriostatic agents, etc. can be added as needed. Furthermore, diluents, dispersants, surfactants, binders, and lubricants may be added to be formulated into injectable formulations such as aqueous solutions, suspensions, emulsions, etc. In particular, it is preferred to formulate and provide the formulation in lyophilized form. For the preparation of lyophilized formulations, common methods known in the art to which the present disclosure belongs may be used, and stabilizing agents for lyophilization may be added.

The method of administration of the pharmaceutical composition can be determined by a person of ordinary skill in the art based on the symptoms of a common patient and the severity of the disease. Furthermore, the pharmaceutical composition can be formulated in various forms, such as acids, tablets, capsules, liquids, injections, ointments, and syrups, etc., and can be provided in unit-dose or multi-dose containers, such as sealed ampoules and bottles, etc.

The pharmaceutical composition of the present disclosure can be administered orally or parenterally. The route of administration of the pharmaceutical composition according to the present disclosure are not limited thereto, but may include, for example, oral, intravenous, intramuscular, intra-arterial, intramedullary, intrathecal, intradural, intracardiac, transdermal, subcutaneous, intraperitoneal, enteral, sublingual, or topical administration. The dosage of the pharmaceutical composition according to the present disclosure may vary in a range depending on the patient's weight, age, gender, health status, diet, time, method of administration, rate of excretion, or severity of disease, etc., and may be readily determined by a person of ordinary skill in the art. Furthermore, the pharmaceutical composition of the present disclosure can be formulated into suitable dosage forms for clinical administration using the techniques disclosed herein.

In another aspect, the present disclosure relates to a method of ameliorating or treating a disease caused by HBV infection, including the step of administering the RNAi agent to a subject. Since the compositions included in an ameliorative or treatment method according to the present disclosure are the same as those included in the disclosure described above, the description is equally applicable to the ameliorative or treatment method.

Hereinafter, the present disclosure will be described in more detail through examples. These examples are intended solely to illustrate the present disclosure, and it will be apparent to one of ordinary skill in the art that the scope of the present disclosure is not to be construed as being limited by these examples.

### Example 1: Screening of RNAi-induced double-stranded nucleic acid molecule targeting HBV

### (1) Derivation of HBV asiRNA by evaluation of HBV proliferation inhibitory ability

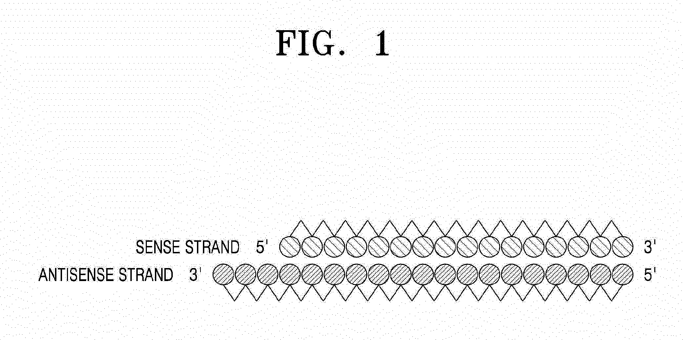

In this example, a hepatitis B virus (HBV) asymmetric siRNA (asiRNA) for screening was designed as a 16 mer (sense strand)-19 mer (antisense strand) asymmetric asiRNA (FIG. 1). In addition, the HBV asiRNA was designed to target the transcripts of the open reading frames (ORFs) of the HBV S or HBV X regions of a HBV genome, that is 3.5 Kb pregenomic RNA, 3.5 Kb precore messenger RNA (mRNA), 2.4 Kb preS1 mRNA, and/or 2.1 Kb pre S2/S mRNA, and 0.7 kb Hbx mRNA, to inhibit their expression.

Thereafter, HBV plasmid and HBV asiRNA were transfected into Hela cells, and the resulting HBV inhibitory ability was confirmed. Specifically, to deliver HBV asiRNA (1 nM) and HBV plasmid (25 ng/well) into Hela cells, lipofectamine 2000 (ThermoFisher, Cat.11668019, 0.25 µl/well) was used. At 24 hours post-transfection, the renilla and firefly luciferase intensity was measured using the dual luciferase reporter assay system (Promega, Cat.E1960), and the level of HBV proliferation inhibition by asiRNA was confirmed by comparing the intensity with that of the group that was delivered with only a HBV plasmid (plasmid only).

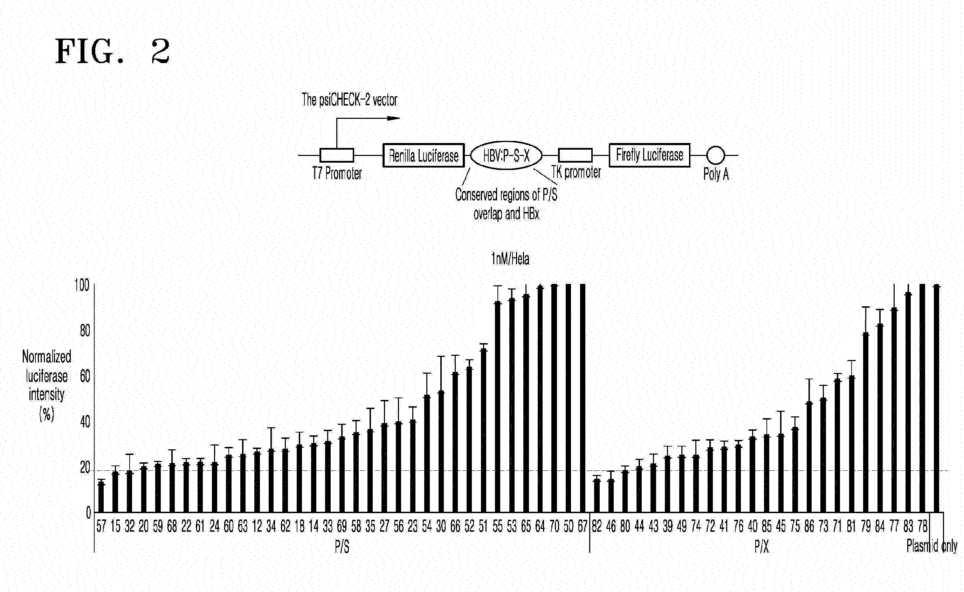

As a result, as shown in FIG. 2, a HBV asiRNA capable of significantly inhibiting HBV proliferation was comfirmed, and the specific sequence information is shown in Table 4.

**Table 4**

| **No.** | **Name** | **Sequence (5' → 3')** | | **SEQ ID NO** |
|---|---|---|---|---|
| 12 | asiHBV-012 | sense | GAUGUGUCUGCGGCGU | 1 |
| | | antisense | ACGCCGCAGACACAUC CAG | 2 |
| 14 | asiHBV-014 | sense | UGUGUCUGCGGCGUUU | 3 |
| | | antisense | AAACGCCGCAGACACAU CC | 4 |
| 18 | asiHBV-018 | sense | GGUAUGUUGCCCGUUU | 5 |
| | | antisense | AAACGGGCAACAUACCU UG | 6 |
| 20 | asiHBV-020 | sense | GCCCGUUUGUCCUCUA | 7 |
| | | antisense | UAGAGGACAAACGGGC AAC | 8 |
| 23 | asiHBV-023 | sense | AGUUUACUAGUGCCAU | 9 |
| | | antisense | AUGGCACUAGUAAACU GAG | 10 |
| 27 | asiHBV-027 | sense | ACUAGUGCCAUUUGUU | 11 |
| | | antisense | AACAAAUGGCACUAGUA AA | 12 |
| 32 | asiHBV-032 | sense | UGUUCAGUGGUUCGUA | 13 |
| | | antisense | UACGAACCACUGAACAA AU | 14 |
| 33 | asiHBV-033 | sense | AGUGGUUCGUAGGGCU | 15 |
| | | antisense | AGCCCUACGAACCACU GAA | 16 |
| 34 | asiHBV-034 | sense | GUGGUUCGUAGGGCUU | 17 |
| | | antisense | AAGCCCUACGAACCACU GA | 18 |
| 35 | asiHBV-035 | sense | UCAGUUAUAUGGAUGA | 19 |
| | | antisense | UCAUCCAUAUAACUGAA AG | 20 |
| 39 | asiHBV-039 | sense | GCACCUCUCUUUACGA | 21 |
| | | antisense | UCGUAAAGAGAGGUGC GCC | 22 |
| 40 | asiHBV-040 | sense | CACCUCUCUUUACGCA | 23 |
| | | antisense | UGCGUAAAGAGAGGUG CGC | 24 |
| 41 | asiHBV-041 | sense | ACCUCUCUUUACGCGA | 25 |
| | | antisense | UCGCGUAAAGAGAGGU GCG | 26 |
| 43 | asiHBV-043 | sense | CCGUCUGUGCCUUCUA | 27 |
| | | antisense | UAGAAGGCACAGACGG GGA | 28 |
| 44 | asiHBV-044 | sense | CGUCUGUGCCUUCUCA | 29 |
| | | antisense | UGAGAAGGCACAGACG GGG | 30 |
| 45 | asiHBV-045 | sense | GUCUGUGCCUUCUCAU | 31 |
| | | antisense | AUGAGAAGGCACAGAC GGG | 32 |
| 46 | asiHBV-046 | sense | GUGCCUUCUCAUCUGA | 33 |
| | | antisense | UCAGAUGAGAAGGCAC AGA | 34 |
| 49 | asiHBV-049 | sense | CCUUCUCAUCUGCCGA | 35 |
| | | antisense | UCGGCAGAUGAGAAGG CAC | 36 |
| 56 | asiHBV-056 | sense | UGGUGGACUUCUCUCA | 37 |
| | | antisense | UGAGAGAAGUCCACCA CGA | 38 |
| 58 | asiHBV-058 | sense | GUCUGCGGCGUUUUAU | 39 |
| | | antisense | AUAAAACGCCGCAGACA CA | 40 |
| 59 | asiHBV-059 | sense | CUGCGGCGUUUUAUCA | 41 |
| | | antisense | UGAUAAAACGCCGCAGA CA | 42 |
| 60 | asiHBV-060 | sense | UGCGGCGUUUUAUCAU | 43 |
| | | antisense | AUGAUAAAACGCCGCAG AC | 44 |
| 61 | asiHBV-061 | sense | CUGCUGCUAUGCCUCA | 45 |
| | | antisense | UGAGGCAUAGCAGCAG GAU | 46 |
| 62 | asiHBV-062 | sense | UGCUGCUAUGCCUCAU | 47 |
| | | antisense | AUGAGGCAUAGCAGCA GGA | 48 |
| 63 | asiHBV-063 | sense | CUAUGCCUCAUCUUCU | 49 |
| | | antisense | AGAAGAUGAGGCAUAG CAG | 50 |
| 68 | asiHBV-068 | sense | UAGUGCCAUUUGUUCA | 51 |
| | | antisense | UGAACAAAUGGCACUAG UA | 52 |
| 69 | asiHBV-069 | sense | UGGUUCGUAGGGCUUU | 53 |
| | | antisense | AAAGCCCUACGAACCAC UG | 54 |
| 72 | asiHBV-072 | sense | CCACGGGGCGCACCUA | 55 |
| | | antisense | UAGGUGCGCCCGUGG UCG | 56 |
| 73 | asiHBV-073 | sense | CACGGGGCGCACCUCU | 57 |
| | | antisense | AGAGGUGCGCCCCGUG GUC | 58 |
| 74 | asiHBV-074 | sense | ACGGGGCGCACCUCUA | 59 |
| | | antisense | UAGAGGUGCGCCCCGU GGU | 60 |
| 75 | asiHBV-075 | sense | CGGGGCGCACCUCUCU | 61 |
| | | antisense | AGAGAGGUGCGCCCCG UGG | 62 |
| 76 | asiHBV-076 | sense | GGGGCGCACCUCUCUU | 63 |
| | | antisense | AAGAGAGGUGCGCCCC GUG | 64 |
| 80 | asiHBV-080 | sense | UCUGUGCCUUCUCAUA | 65 |
| | | antisense | UAUGAGAAGGCACAGA CGG | 66 |
| 82 | asiHBV-082 | sense | UGUGCCUUCUCAUCUA | 67 |
| | | antisense | UAGAUGAGAAGGCACA GAC | 68 |
| 85 | asiHBV-085 | sense | UGCCAACUGGAUCCUA | 69 |
| | | antisense | UAGGAUCCAGUUGGCA GCA | 70 |
| 86 | asiHBV-086 | sense | UGCUGCCAACUGGAUA | 71 |
| | | antisense | UAUCCAGUUGGCAGCA CAG | 72 |

### (2) Evaluation of proliferation inhibitory ability of HBV asiRNA

In this example, 4 types of the HBV asiRNAs derived above (asiHBV-020, asiHBV-032, asiHBV-044, and asiHBV-082) were evaluated for their HBV inhibitory ability in mouse hepatocytes. Specifically, HBV plasmid (20 µg/mouse) was administered to mice by hydrodynamic injection, and hepatocytes were isolated from the mice 6 hours after injection. Thereafter, 4 types of HBV asiRNA were delivered into cells at concentrations of 1 nM and 10 nM each using lipofectamine RNAiMAX (ThermoFisher, Cat. 13778150, 0.1 µl/100 µl). 24 hours after the intracellular delivery, renilla and firefly luciferase intensity were measured using the dual luciferase reporter assay system (Promega, Cat.E1960). The level of inhibition of HBV proliferation by HBV asiRNA was confirmed by comparing the intensities with those of the group treated with only lipofectamine RNAiMAX (Mock). Meanwhile, the control group used was a group delivered with only a HBV plasmid (HDI only).

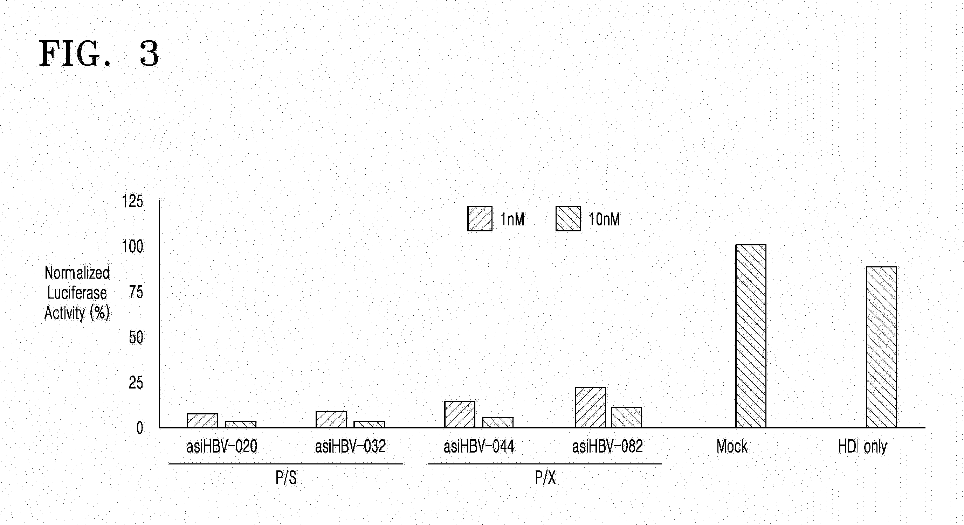

As a result, HBV proliferation inhibitory effect was confirmed according to the treatment with HBV asiRNA (asiHBV-020, asiHBV-032, asiHBV-044, asiHBV-082), as shown in FIG. 3. The effect was significant compared to the group delivered with only the HBV plasmid (HDI only) and treated with only lipofectamine RNAiMAX (Mock), and showed a concentration-dependent trend.

### Example 2: Design and production of HBV asiRNA with chemical modifications introduced

In this example, asymmetric siRNAs with chemical modifications introduced (cell-penetrating-asymmetric siRNA: cp-asiRNA) were designed based on 2 HBV asiRNAs (asiHBV-032 and asiHBV-082) whose proliferation inhibition effects were confirmed in Example 1. The cp-asiRNAs designed in this example is an asymmetric siRNA with various chemical modifications (2'OMe, PS, Fluoro), to enhance delivery into hepatocytes compared to the asiRNAs.

The sequence information of a total of eight cp-asiRNAs produced in this example is shown in Table 5.

**Table 5**

| **No.** | **Name** | **Sequence (5' → 3')** | |
|---|---|---|---|
| 1 | OLX703A-032-1 | sense | mU*fG*mUfUfCfAmGfUmGfGmUfUmCfGmUfA- GalNAc |
| | | antisense | P- mU*fA*mCfGmAfAmCfCmAfCmUmGmAfAmC*fA *mA*fA*mU |
| 2 | OLX703A-032-2 | sense | mU*fG*mUfUfCfAmGfUmGfGmUfUmCfGmUfA- GalNAc |
| | | antisense | P- mU*fA*mCmGmAfAmCfCfAmCmUmGmAfAmC*f A*mA*mA*mU |
| 3 | OLX703A-032-3 | sense | mU*fG*mUfUfCfAmGmUmGmGmUmUmCmGm UmA-GalNAc |
| | | antisense | P- mU*fA*mCfGmAfAmCfCmAfCmUmGmAfAmC*fA *mA*fA*mU |
| 4 | OLX703A-032-4 | sense | mU*fG*mUfUfCfAmGmUmGmGmUmUmCmGm UmA-GalNAc |
| | | antisense | P- mU*fA*mCmGmAfAmCfCfAmCmUmGmAfAmC*f A*mA*mA*mU |
| 5 | OLX703A-082-1 | sense | mU*fG*mUfGfCfCmUfUmCfUmCfAmUfCmUfA- GalNAc |
| | | antisense | P- mU*fA*mGfAmUfGmAfGmAfAmGmGmCfAmC*fA *mG*fA*mC |
| 6 | OLX703A-082-2 | sense | mU*fG*mUfGfCfCmUfUmCfUmCfAmUfCmUfA- GalNAc |
| | | antisense | P- mU*fA*mGmAmUfGmAfGfAmAmGmGmCfAmC*f A*mG*mA*mC |
| 7 | OLX703A-082-3 | sense | mU*fG*mUfGfCfCmUmUmCmUmCmAmUmCmU mA-GalNAc |
| | | antisense | P- mU*fA*mGfAmUfGmAfGmAfAmGmGmCfAmC*fA *mG*fA*mC |
| 8 | OLX703A-082-4 | sense | mU*fG*mUfGfCfCmUmUmCmUmCmAmUmCmU mA-GalNAc |
| | | antisense | P- mU*fA*mGmAmUfGmAfGfAmAmGmGmCfAmC*f A*mG*mA*mC |

The GalNAc is a trivalent GaINAc derivative.

Meanwhile, in Table 5, the structure of the derivative labeled "trivalent GalNAc" coupled to the 3' end of the sense strand is shown in Formula 1.

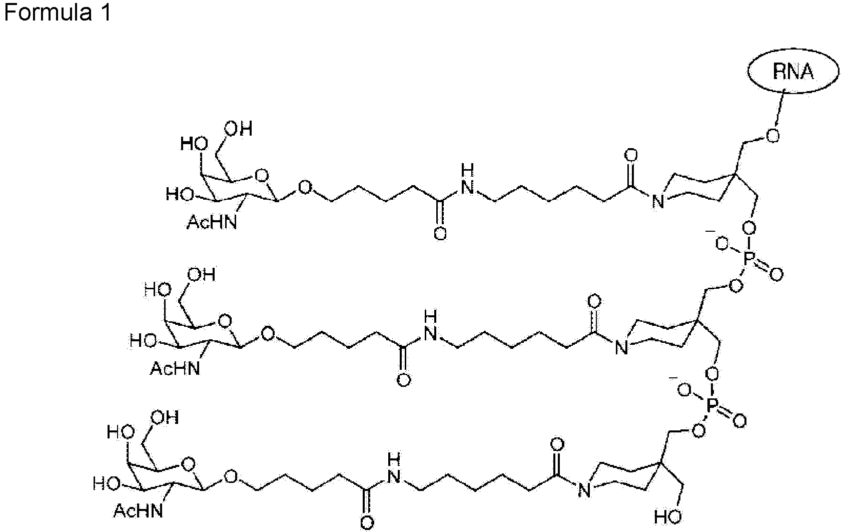

In addition, the chemical modifications labeled "*", "m", "f", "P", and "EVP" in Table 5 are shown in Table 6.

**Table 6**

| **Notation** | **Chemical Modification** |
|---|---|
| * | Phosphorothioate bond |
| m | 2'-O-methyl |
| f | 2'-fluoro |
| P | 5'-phosphate group |
| EVP | 5' E-vinylphosphonate |

Specifically, in Table 6 above, "*" refers to an existing phosphodiester bond substituted with a phosphorothioate bond, and "m" refers to an existing 2'-OH substituted with 2'-O-methyl. In addition, "f" refers to, for example, in the case of fG, the existing 2'-OH of G (guanine) is substituted with a fluoro, "P" refers to a phosphate group linked at the 5'-end, and "EVP" refers to an (E) vinylphosphonate linked at the 5'-end. For example, EVP-mU refers to the 2'-OH of the existing U (uracil) substituted with 2'-O-methyl and an (E) vinylphosphonate linked to the 5'-end.

### Example 3: Evaluation of proliferation inhibitory ability of HBV asiRNA with chemical modifications introduced

In this example, 8 types of HBV cp-asiRNA with chemical modifications introduced in Example 2 were evaluated for their HBV inhibitory ability in mouse hepatocytes. Specifically, HBV plasmid (20 µg/mouse) was administered to mice by hydrodynamic injection, and hepatocytes were isolated from the mice 6 hours after injection. Thereafter, 8 types of HBV cp-asiRNA were delivered into cells at concentrations of 1 nM, 20 nM or 200 nM each using lipofectamine RNAiMAX (ThermoFisher, Cat. 13778150, 0.1 µl/100 µl). 24 hours after the intracellular delivery, renilla and firefly luciferase intensity were measured using the dual luciferase reporter assay system (Promega, Cat.E1960). The level of inhibition of HBV proliferation by HBV cp-asiRNA was confirmed by comparing the intensities with those of the group delivered with only a HBV plasmid (HDI only). Meanwhile, the group treated with only lipofectamine RNAiMAX (Mock) was used as a negative control for the 1 nM transfection group.

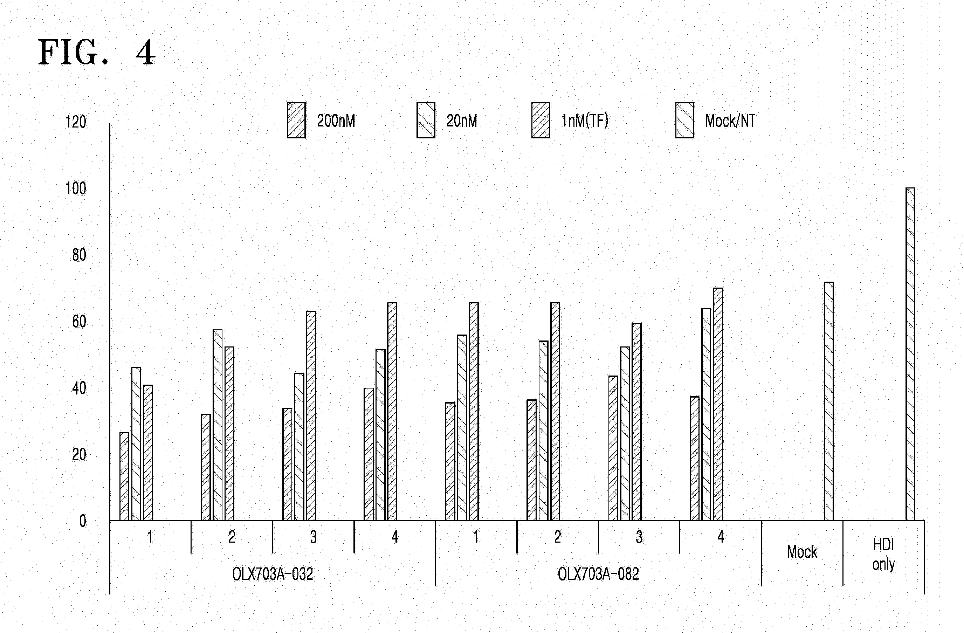

As a result, the inhibition of HBV proliferation by the treatment of 8 types of HBV cp-asiRNA was confirmed, as shown in FIG. 4.

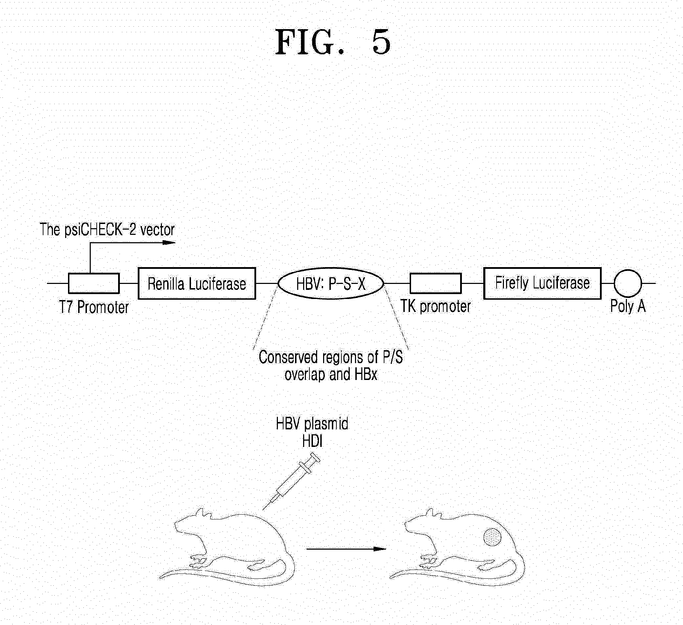

Next, among those with chemical modifications introduced, the HBV inhibitory effects of 2 types of HBV cp-asiRNAs (OLX703A-032-1, OLX703A-082-1) were evaluated, using a mouse model. Specifically, as shown in FIG. 5, an HBV plasmid HDI mouse model was established by administering HBV plasmid (10 µg/mouse) to mice by hydrodynamic injection. In parallel, 2 types of HBV cp-asiRNA were subcutaneously injected into the mouse model at 3 mg/kg or 9 mg/kg, respectively. 48 hours after the subcutaneous injection, renilla and firefly luciferase intensity were measured using a dual luciferase reporter assay system (Promega, Cat.E1960) to confirm the HBV proliferation inhibition level of HBV cp-asiRNAs compared to the group devilered with only a HBV plasmid (Mock control). 3 mice were used for each group, and 2 regions of liver tissue were analyzed for each mouse.

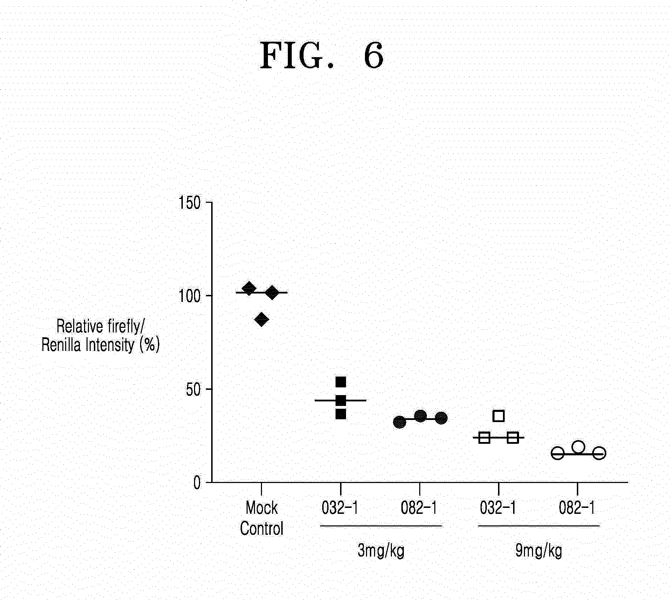

As a result, as shown in FIG. 6, the inhibition of HBV proliferation by treatment with the 2 types of HBV cp-asiRNA was confirmed in vivo. The experimental results indicate that HBV asiRNA/HBV cp-asiRNA according to an embodiment can be used for the treatment of diseases associated with HBV infection.

### Example 4: Additional design and production of HBV asiRNA with chemical modifications introduced

In this example, 6 types HBV asiRNAs (asiHBV-020, asiHBV-032, asiHBV044, asiHBV-046, asiHBV-080, asiHBV-082) among the HBV asiRNAs whose proliferation inhibitory effects were confirmed in Example 1 were further designed as asymmetric siRNA (cell-penetrating-asymmetric siRNA: cp-asiRNA) with chemical modifications introduced. The cp-asiRNAs designed in this example is an asymmetric siRNA with various chemical modifications, to enhance delivery into hepatocytes compared to the asiRNAs.

In addition, in the following examples to confirm the effects of the HBV asiRNA and cp-asiRNA of the present disclosure, a cp-asiRNA (OLX700A-001-8) targeting the Factor 9 gene was used as a negative control (NC).

The sequence information of a total of 19 types of cp-asiRNA produced in this example is listed in Table 7, and the sequence information of the NC is listed in Table 8. (* GalNAc is a trivalent GalNAc derivative).

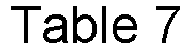

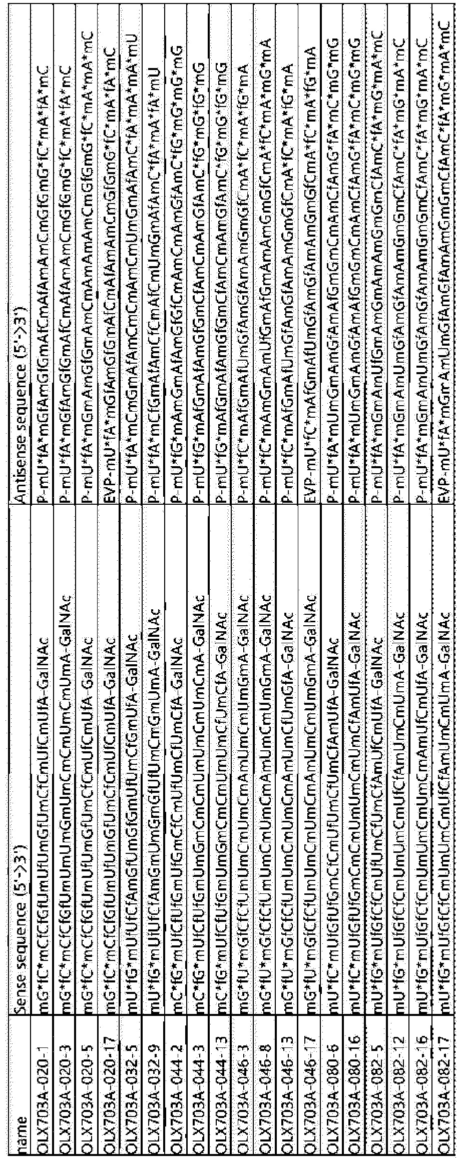

**Table 8**

| **Name** | **Sequence (5' → 3')** | |
|---|---|---|
| OLX700A-001-8 (NC) | sense | mG*fC*mAfGfUfAmUfGmUfUmGfAmUfGmGfA- GalNAc |
| | antisens e | P- mU*fC*mCfAmUfCmAfAmCfAmUmAmCfUmG*fC* mU*fU*mC |

Meanwhile, in Table 7 and Table 8, the structure of GalNAc linked to the 3' end of the sense strand is as shown in Formula 1. In addition, the chemical modifications labeled "*", "m", "f", "P", and "EVP" in Table 7 and Table 8 are as shown in Table 6.

### Example 5: Evaluation of proliferation inhibitory ability of HBV asiRNA with chemical modifications introduced

### (1) Evaluation of HBV proliferation inhibitory ability in vivo

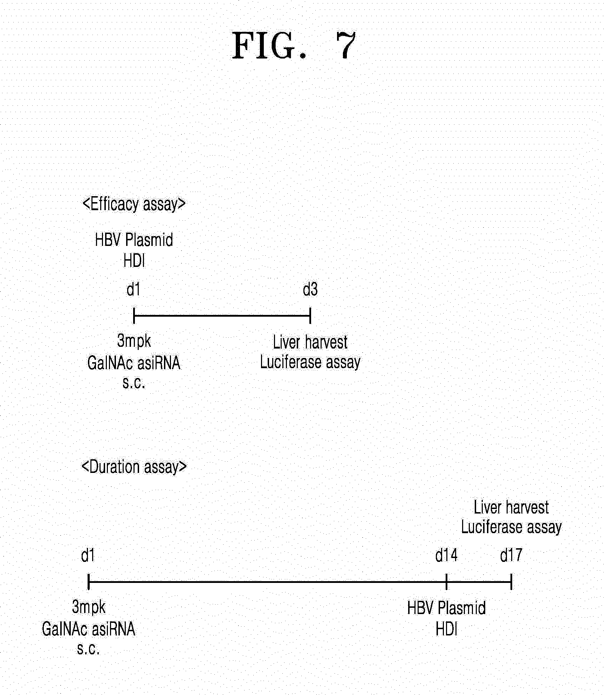

In this example, HBV inhibitory ability was evaluated by efficacy assay and duration assay for HBV cp-asiRNAs with chemical modifications introduced in Examples 2 and 4 (FIG. 7).

First, for the efficacy assay, HBV plasmid (20 µg/mouse) was administered into the tail vein of BALB/c mice by hydrodynamic injection. Each GalNAc-asiRNA was injected subcutaneously between the shoulder blades at a concentration of 3 mg/kg. 3 days later, 2 regions of mouse liver tissue (left lobe, caudate lobe) were obtained, placed in 1 ml of 1X protein lysis buffer, and crushed with a tissue homogenizer. Centrifugation was performed at 13,000 rpm at 4 °C for 15 minutes, and transferred only the supernatant to a new tube. Renilla and firefly luciferase intensity was measured using a dual luciferase reporter assay system (Promega, Cat.E1960). The degree of HBV inhibition of GaINAc-asiRNA was confirmed by comparing the intensity with that of the group delivered with only a HBV plasmid (control group).

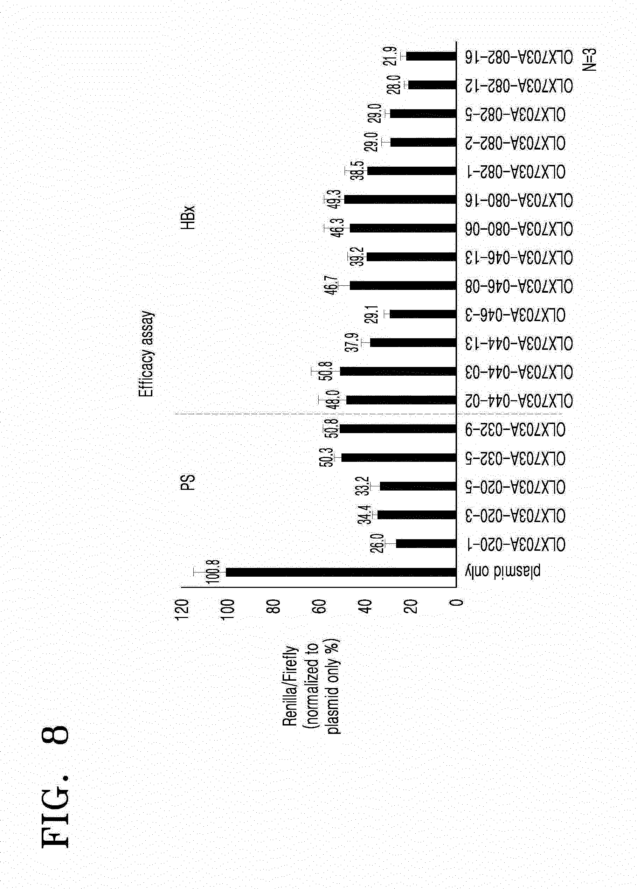

As a result, shown in FIG. 8, the HBV proliferation inhibitory effect was confirmed according to the treatment of 18 types of HBV cp-asiRNA.

Next, for the duration assay, each GalNAc-asiRNA was injected subcutaneously between the shoulder blades at a concentration of 3 mg/kg, and 14 days later, HBV plasmid (20 µg/mouse) was administered into the tail vein by hydrodynamic injection. 3 days later, 2 regions of mouse liver tissue (left lobe, caudate lobe) were obtained, placed in 1 ml of 1X protein lysis buffer, and crushed with a homogenizer. Centrifugation was performed at 13,000 rpm at 4 °C for 15 minutes, and transferred only the supernatant to a new tube. Renilla and firefly luciferase intensity was measured using the dual luciferase reporter assay system (Promega, Cat.E1960). The degree of HBV inhibition of GaINAc-asiRNA was confirmed by comparing the intensity with that of the group delivered with only a HBV plasmid (control group).

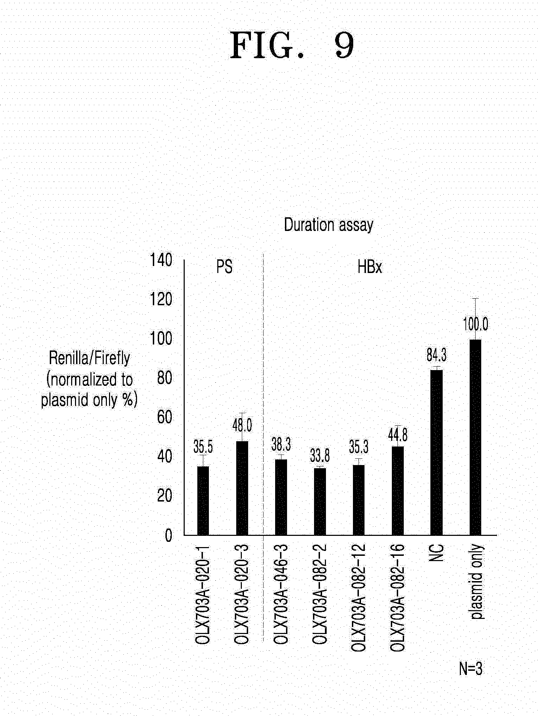

As a result, as shown in FIG. 9, it was confirmed that the HBV proliferation inhibitory effect according to the treatment of 6 types of HBV cp-asiRNA, and confirmed that the HBV cp-asiRNA can show stable activity for a long time in vivo.

### (2) Evaluation of HBV proliferation inhibitory ability in vitro

In this example, 6 types of the HBV cp-asiRNAs with chemical modifications introduced in Examples 2 and 4 were evaluated for HBV inhibitory ability at the cellular level (in vitro).

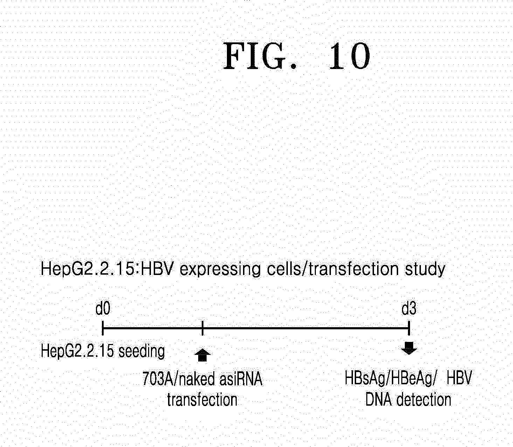

Specifically, experiments were performed using the HBV-expressing HepG2.2.15 cell line (FIG. 10), and 3 types of asiRNA (asiHBV-020, asiHBV-046, asiHBV-082) and their GalNAc-asiRNA were used. The siRNAs were delivered into cells at a concentration of 10 nM using lipofectamine RNAiMAX (ThermoFisher, Cat. 13778150, 4 ml/well). After 3 days of cultivation, the levels of HBsAg and HBeAg were confirmed in the supernatant by enzyme-linked immunosorbent assay (ELISA), and the expression levels of HBV DNA were determined in the cells by quantitative polymerase chain reaction (qPCR).

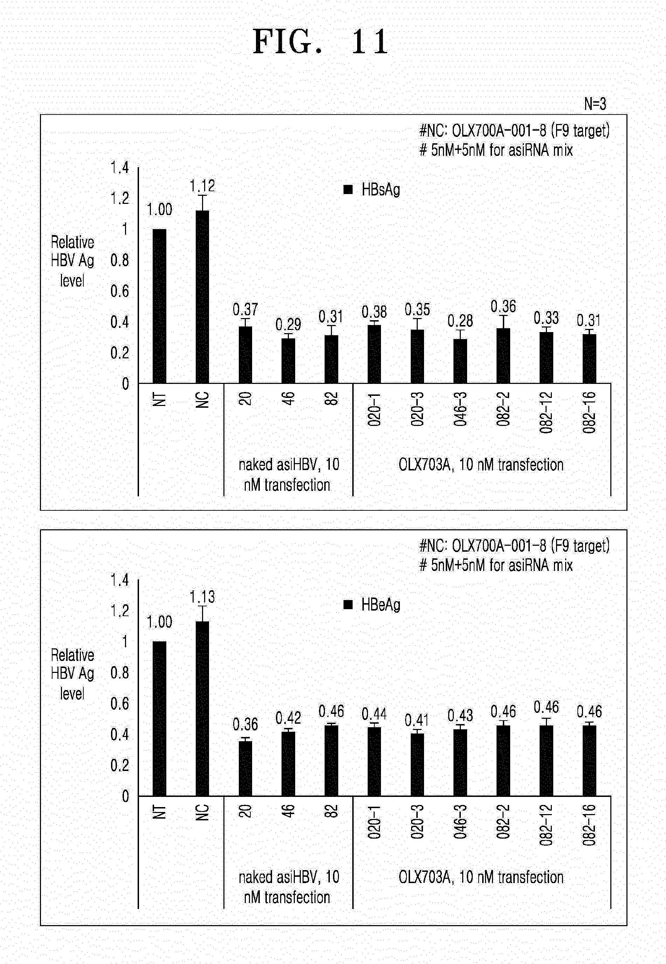

As a result, as shown in FIG. 11, it was confirmed that both the HBV asiRNAs targeting P/S and P/S/X and their GalNAc-asiRNAs effectively inhibit HBV.

### (3) Evaluation of HBV proliferation inhibitory ability using AAV-HBV mouse model

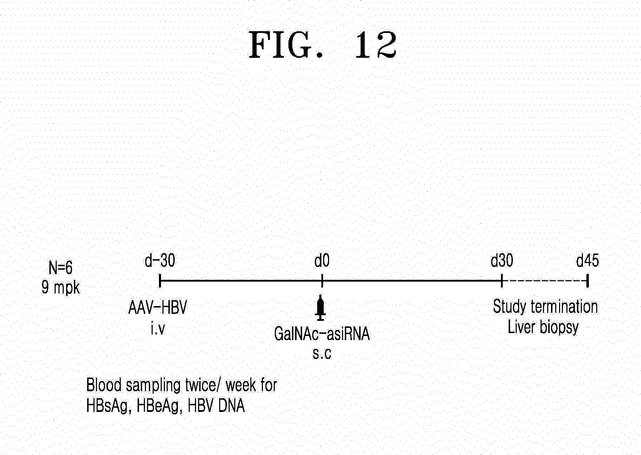

In this example 3 types of HBV cp-asiRNAs with chemical modifications introduced in Example 4 were evaluated for HBV inhibitory ability using an efficacy assay in an AAV-HBV mouse model (FIG. 12).

Specifically, the efficacy assay in the AAV-HBV mouse model was performed at WuXi AppTec Co., Ltd. 30 days prior to substance administration, AAV-HBV mouse models were set up by intravenous injection (i.v injection) of AAV-HBV 1.0×10¹¹ viral titer into C57B/L6 mice. On Day 0 of substance administration, GalNAc-asiRNA was administered to the mouse model by subcutaneous injection at a dose of 9 mg/kg. Blood was collected twice/week for a total of 28 days, and the levels of HBsAg and HBeAg in the blood were measured by enzyme-linked immunosorbent assay (ELISA), and the expression levels of HBV DNA were measured by quantitative polymerase chain reaction (qPCR). After 30 days of substance administration, liver tissues were obtained from the mice and HBsAg was confirmed by IHC.

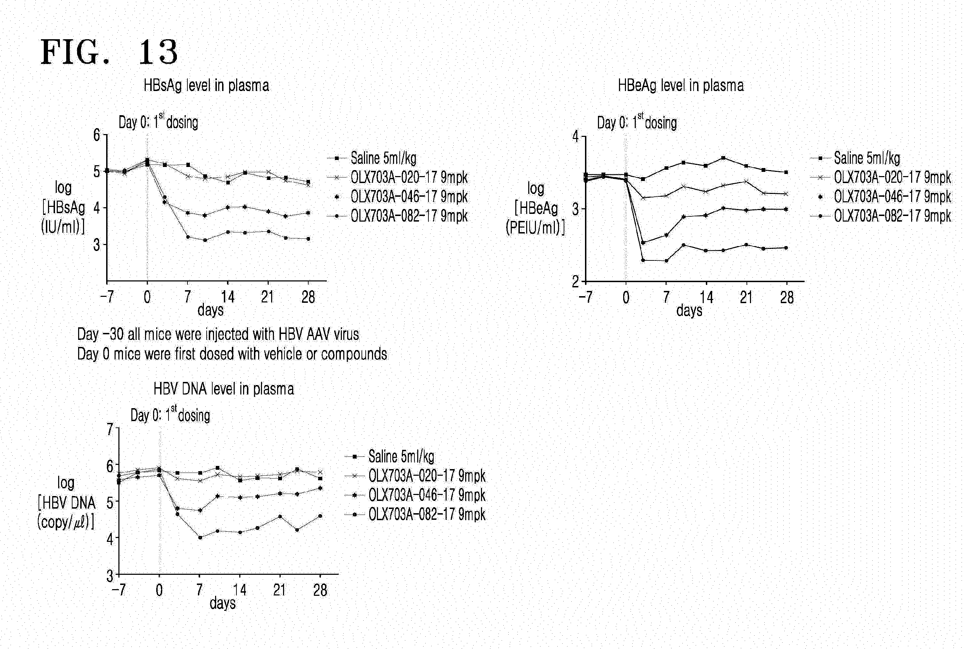

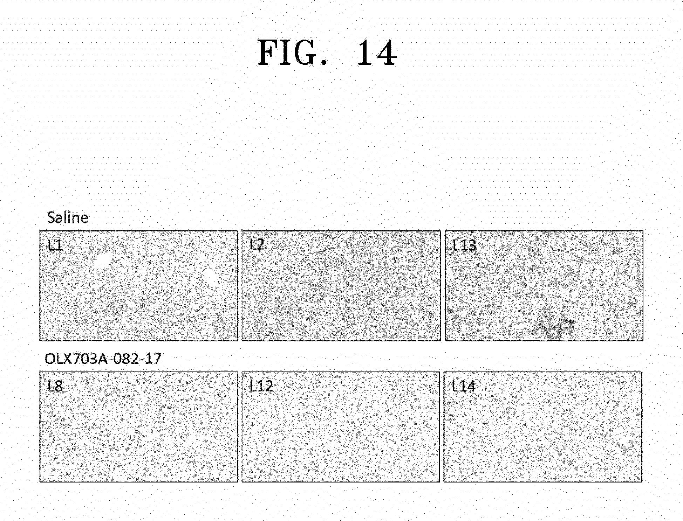

As a result, as shown in FIG. 13, it was confirmed that the 3 types of HBV cp-asiRNA treated could effectively inhibit HBV proliferation for a long time. In addition, mouse liver tissues were obtained and IHC was performed, as shown in FIG. 14, it was confirmed that HBsAg was expressed in the cytoplasm or cell membrane in the saline group, but the expression was significantly reduced in the tissues treated with HBV cp-asiRNAs.

### Example 6: Improvement of RNAi-induced double-stranded nucleic acid molecule targeting HBV

In this exampleHBV asiRNA was produced by modifying the length of the sense or antisense sequence of asiHBV-082, which showed excellent HBV proliferation inhibitory ability in Example 5, and the specific sequence information is shown in Table 9.

**Table 9**

| **No.** | **Name** | **Sequence (5' → 3')** | | **SEQ ID NO** |
|---|---|---|---|---|
| 82 | asiHBV-082 | sense | UGUGCCUUCUCAUCUA | 67 |
| | | antisense | UAGAUGAGAAGGCACA GAC | 68 |
| 103 | asiHBV-103 | sense | UGUGCCUUCUCAUCUA | 73 |
| | | antisense | UAGAUGAGAAGGCACA GACGG | 74 |
| 107 | asiHBV-107 | sense | CUGUGCCUUCUCAUCU A | 75 |
| | | antisense | UAGAUGAGAAGGCACA GACGG | 76 |

Next, asymmetric siRNA (cell-penetrating-asymmetric siRNA: cp-asiRNA) with chemical modifications introduced were designed based on the HBV asiRNAs. The cp-asiRNAs designed in this example is an asymmetric siRNA with various chemical modifications, to enhance delivery into hepatocytes compared to the asiRNAs.

The sequence information of a total of 22 types of cp-asiRNA produced in this example is shown in Table 10 (* GalNAc is a trivalent GalNAc derivative).

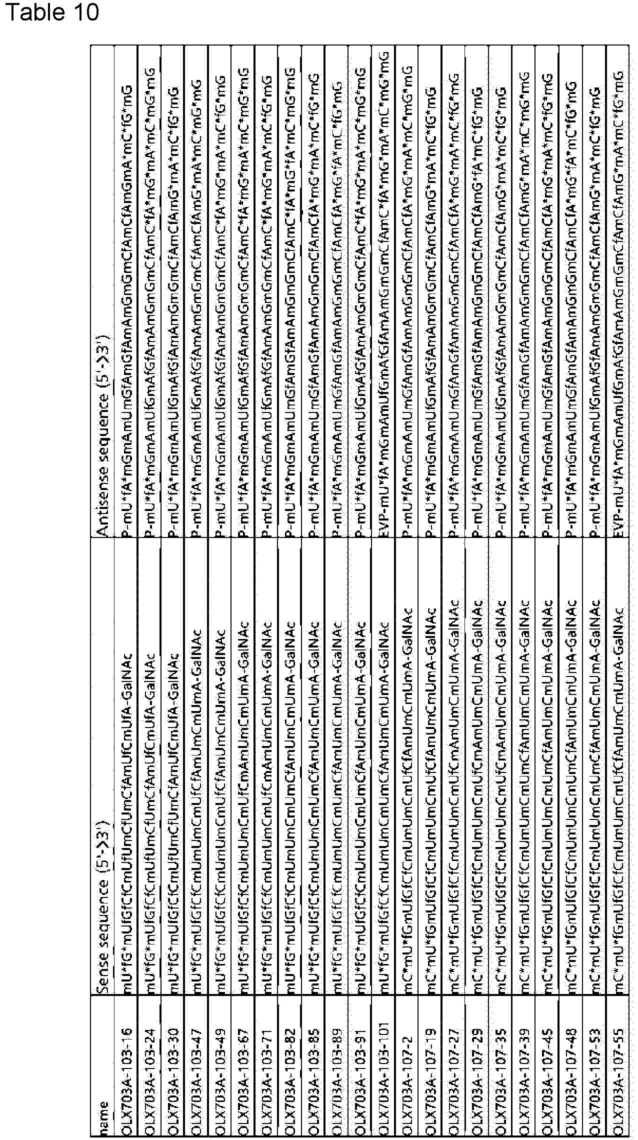

Meanwhile, in Table 10, the structure of N-acetyl-galactosamine (GaINAc) linked to the 3' end of the sense strand is as shown in Formula 1. In addition, the chemical modifications indicated by "*", "m", "f", "P" and "EVP" in Table 9 are as shown in Table 6.

### Example 7: Evaluation of proliferation inhibitory ability of HBV asiRNA with chemical modifications introduced

### (1) Evaluation of HBV proliferation inhibitory ability in vitro

In this example, 20 types of HBV cp-asiRNAs with chemical modifications introduced in Example 6 were evaluated for HBV inhibitory ability at the cellular level (in vitro).

Specifically, hepatocytes were isolated from C57B/L6 mice and seeded with 1×10⁴ cells/well, then treated with two concentrations (200 nM, 20 nM) of GalNAc-asiRNA in the form of 16/21 (asiHBV-103) and 17/21 (asiHBV-107). After 24 hours, 50 ng of HBV plasmid was delivered into cells using lipofectamine 2000 (ThermoFisher, Cat.11668019, 0.2 µl/well). After 24 hours, the medium was replaced with a maintenance medium and cultured for another 24 hours, and renilla and firefly luciferase intensity were measured using the dual luciferase reporter assay system (Promega, Cat.E1960). The degree of HBV inhibition of GalNAc-asiRNA was confirmed by comparing the intensity with that of the NC-treated control group.

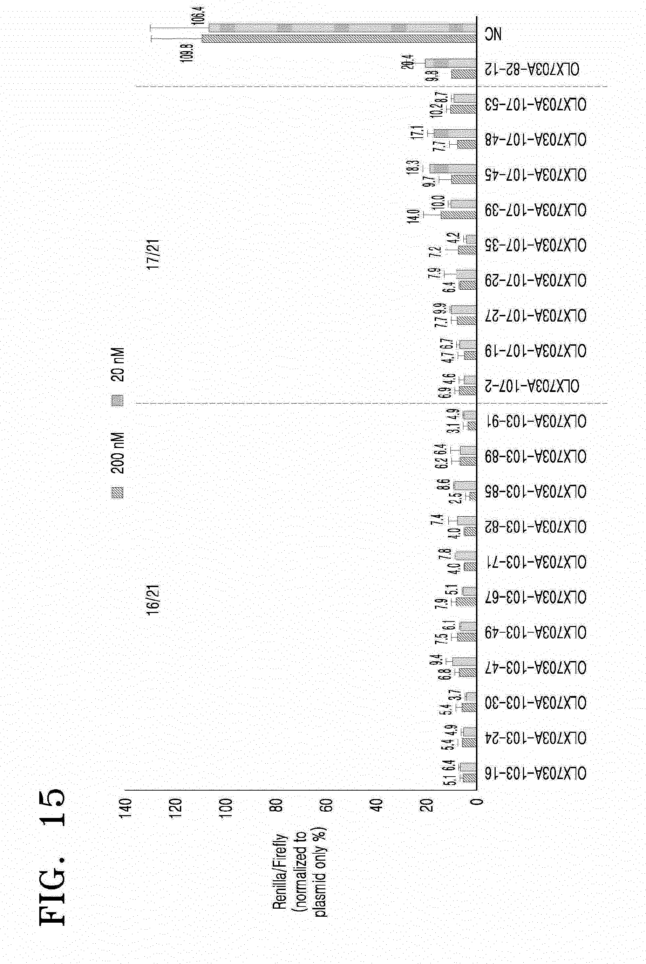

As a result, as shown in FIG. 15, the HBV proliferation inhibitory effect according to the treatment of 20 types of HBV cp-asiRNA was confirmed.

### (2) Evaluation of HBV proliferation inhibitory ability in vivo

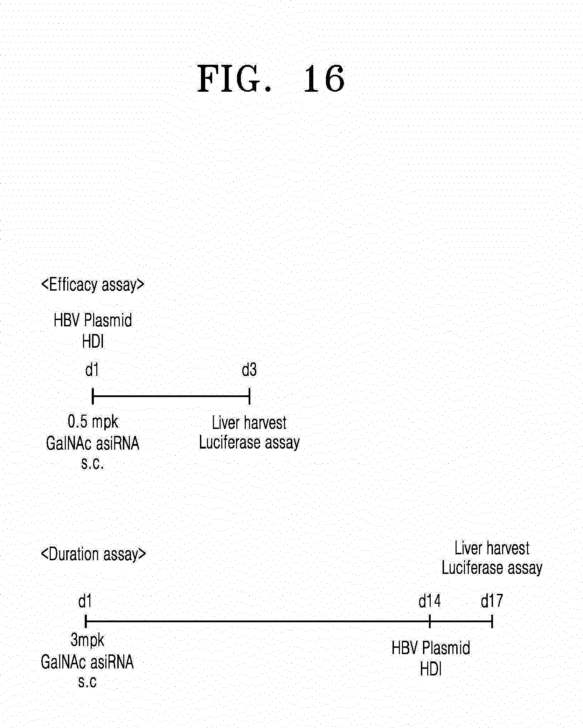

In this example HBV inhibitory ability was evaluated using the efficacy assay and duration assay for either 6 types or 3 types of HBV cp-asiRNAs with chemical modifications introduced in Example 6 (FIG. 16).

First, for the efficacy assay, HBV plasmid (10 µg/mouse) was administered into the tail vein of BALB/c mice by hydrodynamic injection. Each GalNAc-asiRNA was injected subcutaneously between the shoulder blades at a concentration of 0.5 mg/kg. 3 days later, 2 regions of mouse liver tissue (left lobe, caudate lobe) were obtained, placed in 0.5 ml of 1X protein lysis buffer, and crushed with a tissue homogenizer. Centrifugation was performed at 13,000 rpm at 4 °C for 15 minutes, and transferred only the supernatant to a new tube. Renilla and firefly luciferase intensity was measured using a dual luciferase reporter assay system (Promega, Cat.E1960). The degree of HBV inhibition of GaINAc-asiRNA was confirmed by comparing the intensity with that of the group delivered with only a HBV plasmid (control group).

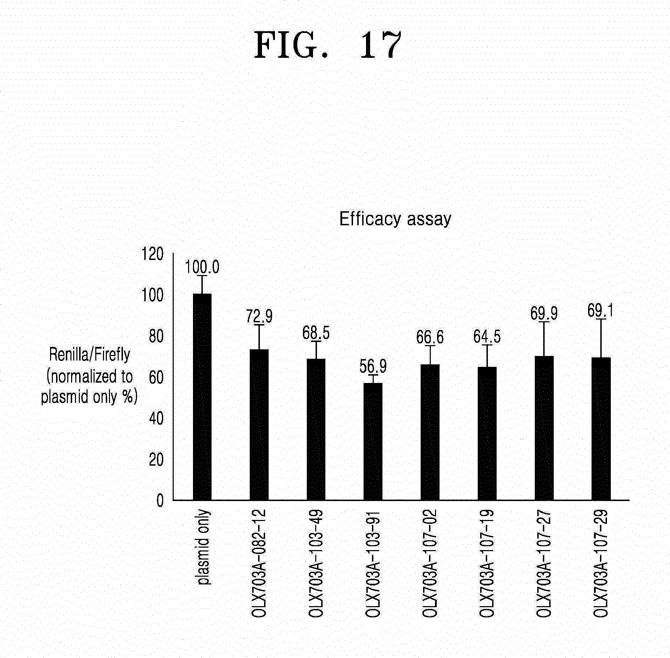

As a result, as shown in FIG. 17, it was confirmed that 7 types of HBV cp-asiRNA had excellent HBV proliferation inhibitory effects.

Next, for the duration assay, each GalNAc-asiRNA was injected subcutaneously between the shoulder blades at a concentration of 1 mg/kg, and 14 days later, HBV plasmid (10 µg/mouse) was administered by hydrodynamic injection into the tail vein. 3 days later, 2 regions of mouse liver tissue (left lobe, caudate lobe) were obtained, placed in 0.5 ml of 1X protein lysis buffer, and crushed with a tissue homogenizer. Centrifugation was performed at 13,000 rpm at 4 °C for 15 minutes, and transferred only the supernatant to a new tube. Renilla and firefly luciferase intensity was measured using the dual luciferase reporter assay system (Promega, Cat.E1960). The degree of HBV inhibition of GalNAc-asiRNA was confirmed by comparing the intensity with that of the group delivered with only a HBV plasmid (control group).

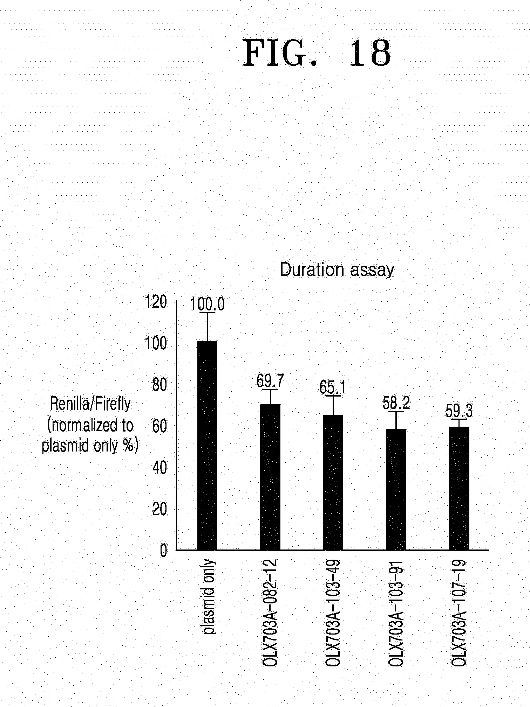

As a result, as shown in FIG. 18, it was confirmed that the 4 types of HBV cp-asiRNA can exhibit excellent HBV proliferation inhibitory efficacy for a long time in vivo.

### (3) IC50 Evaluation

In this example, the IC50 of 3 types of HBV asiRNA (OLX703A-83, OLX703A103, and OLX703A-107) were evaluated in comparison.

Specifically, the Hela cell line 8×10³/well was seeded in 96-well plates, and the next day co-transfected with 25 ng of HBV plasmid at concentrations of 0.001 nM to 3 nM of each substance using lipofectamine 2000 (0.2 µl/100 ml). After 24 hours of culturing, the medium was replaced with a fresh medium and cultured for another 24 hours. Renilla and firefly luciferase intensity were measured using the Dual luciferase report assay system (Promega, Cat.E1960) and compared to the intensity in the group delivered with only a HBV plasmid (control).

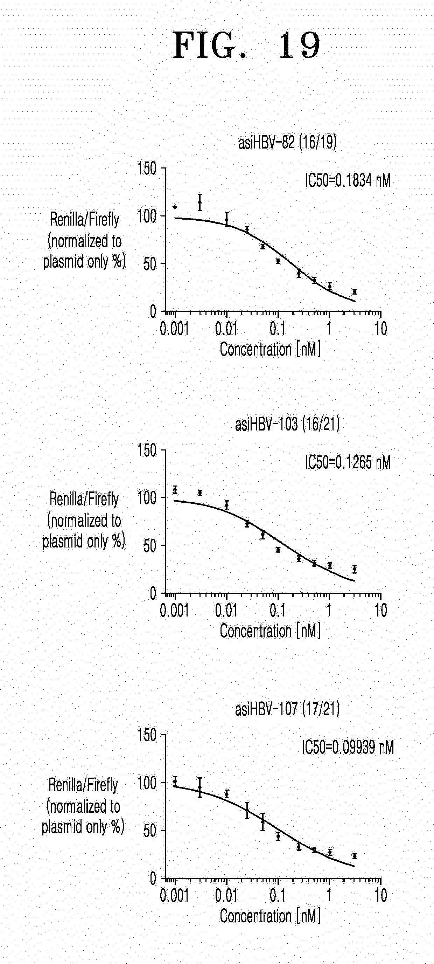

As a result, as shown in FIG. 19, it was confirmed that the 3 types of cp-asiRNA exhibited significantly low IC50.

### Example 8: Evaluation of proliferation inhibitory ability of HBV asiRNA with E-VP modification introduced

In this example, the HBV inhibitory ability of a variant with an additional E-vinylphosphonate (E-VP) at the 5' end of an antisense sequence of a HBV cp-asiRNA with chemical modifications introduced was evaluated.

Specifically, an E-VP modification was introduced at the 5' end of the antisense sequences of OLX703A-082-12, OLX703A-103-91 and OLX703A-107-19, which was named OLX703A-082-17, OLX703A-103-101 and OLX703A-107-55, respectively. Next, HBV plasmid (20 µg/mouse) was administered into the tail vein of BALB/c mice by hydrodynamic injection. Each GalNAc-asiRNA was injected subcutaneously between the shoulder blades at different concentrations (3 mg/kg, 9 mg/kg). 3 days later, 2 regions of mouse liver tissue (left lobe, caudate lobe) were obtained, placed in 0.5 ml of 1X protein lysis buffer, and crushed with a tissue homogenizer. Renilla and firefly luciferase intensity were measured using the dual luciferase reporter assay system (Promega, Cat.E1960). The degree of HBV inhibition of GalNAc-asiRNA was confirmed by comparing the intensity with that of NC.

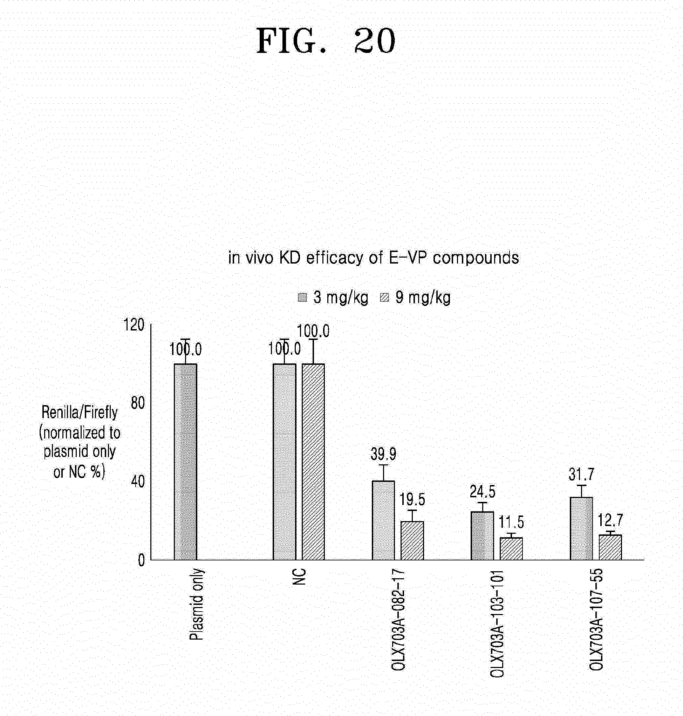

As a result, as shown in FIG. 20, the 3 types of HBV cp-asiRNA with EVP were confirmed to have excellent HBV proliferation inhibitory effects.

The specific aspects of the present disclosure have been described in detail, and such specific descriptions are merely illustrative embodiments to those skilled in the art, and do not limit the scope of the present disclosure. It will be apparent to those skilled in the art that these specific descriptions are merely exemplary and that the scope of the present disclosure is not limited thereby. Accordingly, the substantial scope of the present disclosure is defined by the appended claims and their equivalents.

## Claims

1. An RNA interference (RNAi) agent for inhibiting the expression of hepatitis B virus(HBV), comprising a sense strand and an antisense strand, wherein
the antisense strand has a length of 19 nt to 23 nt,
the sense strand has a length of 15 nt to 17 nt, forms a complementary bond with the antisense strand, comprises a sequence selected from the group consisting of SEQ ID NOs: 1, 3, 5, 7, 9, 11, 13, 15, 17, 19, 37, 39, 41, 43, 45, 47, 49, 51, and 53, and comprises a polyvalent galactose or N-acetyl-galactosamine derivative, and
the 5' end of the antisense strand and the 3' end of the sense strand form a blunt end.

2. The RNAi agent of claim 1, wherein the antisense strand comprises a sequence selected from the group consisting of SEQ ID NOs: 2, 4, 6, 8, 10, 12, 14, 16, 18, 20, 38, 40, 42, 44, 46, 48, 50, 52, and 54.

3. The RNAi agent of claim 1 or claim 2, wherein the sense strand comprises a trivalent N-acetyl-galactosamine derivative.

4. The RNAi agent of claim 1 or claim 2, wherein the sense strand or the antisense strand of the RNAi agent comprises one or more chemical modifications selected from the group consisting of:
substitution of the -OH group at the 2' carbon position of a sugar structure in a nucleotide with methyl (-CH₃), methoxy (-OCH₃), -NH₂ , fluoro (-F), -O-2-methoxyethyl-O-propyl, -O-2-methylthioethyl, -O-3-aminopropyl, or -O-3-dimethylaminopropyl;
substitution of oxygen in a sugar structure in a nucleotide with sulfur;
modification of a nucleotide bond to a phosphorothioate, boranophosphate, or methyl phosphonate;
modification to a peptide nucleic acid (PNA), a locked nucleic acid (LNA), or an unlocked nucleic acid (UNA) form; and
linkage to a phosphate group, E-vinylphosphonate, or a cell-penetrating peptide.

5. An RNA interference (RNAi) agent for inhibiting the expression of hepatitis B virus, comprising a sense strand and an antisense strand, wherein
the antisense strand has a length of 19 nt to 23 nt,
the sense strand has a length of 15 nt to 17 nt, forms a complementary bond with the antisense strand, comprises a sequence selected from the group consisting of SEQ ID NOs: 21, 23, 25, 27, 29, 31, 33, 35, 55, 57, 59, 61, 63, 65, 67, 69, 71, 73, and 75, and comprises a polyvalent galactose or N-acetyl-galactosamine derivative, and
the 5' end of the antisense strand and the 3' end of the sense strand form a blunt end.

6. The RNAi agent of claim 5, wherein the antisense strand comprises a sequence selected from the group consisting of SEQ ID NOs: 22, 24, 26, 28, 30, 32, 34, 36, 56, 58, 60, 62, 64, 66, 68, 70, 72, 74, and 76.

7. The RNAi agent of claim 5 or claim 6, wherein the sense strand comprises a trivalent N-acetyl-galactosamine derivative.

8. The RNAi agent of claim 5 or claim 6, wherein a sense strand or an antisense strand of the RNAi agent comprises one or more chemical modifications selected from the group consisting of:
substitution of the -OH group at the 2' carbon position of a sugar structure in a nucleotide with methyl (-CH₃), methoxy (-OCH₃), -NH₂ , fluoro (-F), -O-2-methoxyethyl-O-propyl, -O-2-methylthioethyl, -O-3-aminopropyl, or -O-3-dimethylaminopropyl;
substitution of oxygen in a sugar structure in a nucleotide with sulfur;
modification of the nucleotide bond to a phosphorothioate, boranophosphate, or methyl phosphonate;
modification to peptide nucleic acid (PNA), locked nucleic acid (LNA), or unlocked nucleic acid (UNA) form; and
linkage to a phosphate group, E-vinylphosphonate, or a cell-penetrating peptide.

9. An RNA interference (RNAi) agent for inhibiting hepatitis B virus, comprising a sense strand and an antisense strand, wherein the antisense strand comprises at least one selected from the group consisting of (a) through (m),
(a) P-mU*fA*mCfGmAfAmCfCmAfCmUmGmAfAmC*fA*mA*fA*mU;
(b) P-mU*fA*mCmGmAfAmCfCfAmCmUmGmAfAmC*fA*mA*mA*mU;
(c) P-mU*fA*mGfAmUfGmAfGmAfAmGmGmCfAmC*fA*mG*fA*mC;
(d) P-mU*fA*mGmAmUfGmAfGfAmAmGmGmCfAmC*fA*mG*mA*mC;
(e) P-mU*fA*mGfAmGfGmAfCmAfAmAmCmGfGmG*fC*mA*fA*mC;
(f) P-mU*fC*mAfGmAfUmGfAmGfAmAmGmGfCmA*fC*mA*fG*mA;
(g) P-mU*fA*mGmAmUmGfAmGfAmAmGmGmCfAmC*fA*mG*mA*mC;
(h) P-mU*fA*mGmAmUmGfAmGfAmAmGmGmCfAmC*fA*mG*mA*mC;
(i) EVP-mU*fA*mGmAmUmGfAmGfAmAmGmGmCfAmC*fA*mG*mA*mC;
(j) P-mU*fA*mGmAmUfGmAfGfAmAmGmGmCfAmC*fA*mG*mA*mC*mG*mG;
(k) EVP-mU*fA*mGmAmUfGmAfGfAmAmGmGmCfAmC*fA*mG*mA*mC*mG*mG;
(l) P-mU*fA*mGmAmUfGmAfGfAmAmGmGmCfAmCfAmG*mA*mC*fG*mG; and
(m) EVP-mU*fA*mGmAmUfGmAfGfAmAmGmGmCfAmCfAmG*mA*mC*fG*mG;
wherein * refers to modification to a phosphorothioate linkage, m refers to substitution with 2'-OCH₃ , f refers to substitution with 2'-F, P refers to a 5'-phosphate group linkage, and EVP refers to f 5'-E-vinylphosphonate linkage.

10. An RNA interference (RNAi) agent for inhibiting hepatitis B virus, comprising a sense strand and an antisense strand, wherein the sense strand comprises at least one selected from the group consisting of (A) through (M),
(A) m U*fG*m UfUfCfAmGfUmGfGm UfUmCfGm UfA-Gal NAc;
(B) mU*fG*mUfUfCfAmGmUmGmGmUmUmCmGmUmA-GaINAc;
(C) mU*fG*m UfGfCfCm UfUmCfUmCfAm UfCm UfA-Gal NAc;
(D) mU*fG*mUfGfCfCmUmUmCmUmCmAmUmCmUmA-GalNAc;
(E) mG*fC*mCfCfGfUmUfUmGfUmCfCmUfCmUfA-GalNAc;
(F) mG*fU*mGfCfCfUmUmCmUmCmAmUmCmUmGmA-GaINAc;
(G) mU*fG*mUfGfCfCmUmUmCmUfCfAmUmCmUmA-GaINAc;
(H) mU*fG*mUfGfCfCmUmUmCmUmCmAmUfCmUfA-GaINAc;
(I) mU*fG*mUfGfCfCmUmUmCmUfCfAmUmCmUmA-GaINAc;
(J) mU*fG*mUfGfCfCmUmUmCmUmCfAmUmCmUmA-GaINAc;
(K) m U*fG*m UfGfCfCmUm UmCm UmCfAmUmCm UmA-Gal NAc;
(L) mC*mU*fGmUfGfCfCmUmUmCmUfCfAmUmCmUmA-GalNAc; and
(M) mC*m U*fGm UfGfCfCm Um UmCm UfCfAm UmCm UmA-Gal NAc;
wherein * refers to modification to a phosphorothioate linkage, m refers to substitution with 2'-OCH₃, f refers to substitution with 2'-F, and GalNAc refers to a trivalent N-acetyl-galactosamine derivative linked to the 3'-end.

11. A pharmaceutical composition for ameliorating or treating a disease caused by hepatitis B virus infection, comprising, as an active ingredient, the RNA interference (RNAi) agent according to any one of claims 1, 2, 5, 6, 9, and 10.
